(19) Europäisches Patentamt
European Patent Office
Office européen des brevets

(11) Veröffentlichungsnummer : **0 058 341**
**B1**

(12) # EUROPÄISCHE PATENTSCHRIFT

(45) Veröffentlichungstag der Patentschrift : **15.05.85**

(21) Anmeldenummer : **82100745.7**

(22) Anmeldetag : **03.02.82**

(51) Int. Cl.[4] : **C 07 D487/04**, C 07 D491/048, C 07 D495/04, A 61 K 31/55 // (C07D487/04, 223:00, 209:00),(C07D491/048, 223:00, 307:00),(C07D495/04, 223:00, 333:00)

(54) **Azepinderivate, ihre Herstellung und diese enthaltende Arzneimittel.**

(30) Priorität : **18.02.81 DE 3105858**

(43) Veröffentlichungstag der Anmeldung : **25.08.82 Patentblatt 82/34**

(45) Bekanntmachung des Hinweises auf die Patenterteilung : **15.05.85 Patentblatt 85/20**

(84) Benannte Vertragsstaaten : **AT BE CH DE FR IT LI LU NL SE**

(56) Entgegenhaltungen :
**US-A- 3 758 501**
**US-A- 3 846 446**
**US-A- 3 849 441**

(73) Patentinhaber : **Dr. Karl Thomae GmbH**
**Postfach 1755**
**D-7950 Biberach (Riss) (DE)**

(72) Erfinder : **Sauter, Robert, Dr. Dipl.-Chem.**
**Albert-Schweizer-Weg 9**
**D-7958 Laupheim (DE)**
Erfinder : **Griss, Gerhart, Dr. Dipl.-Chem.**
**Schopperweg 1**
**D-7950 Biberach 1 (DE)**
Erfinder : **Grell, Wolfgang, Dr. Dipl.-Chem.**
**Amriswilstrasse 7**
**D-7950 Biberach 1 (DE)**
Erfinder : **Hurnaus, Rudolf, Dr. Dipl.-Chem.**
**Silcherstrasse 19**
**D-7950 Biberach 1 (DE)**
Erfinder : **Eisele, Bernhard, Dr. Dipl.-Chem.**
**Beethovenstrasse 12**
**D-7950 Biberach 1 (DE)**
Erfinder : **Haarmann, Walter, Dr.**
**Schlierholzweg 27**
**D-7950 Biberach 1 (DE)**
Erfinder : **Rupprecht, Eckhard, Dr. Dipl.-Biologe**
**Riedbachstrasse 15**
**D-7960 Aulendorf-Tannhausen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 058 341 B1

Jouve, 18, rue St-Denis, 75001 Paris, France

**Beschreibung**

Im US-Patent 3 758 501 werden bereits Tetrahydropyrrolo-, Furo- und Thieno [3,2-c] azepine beschrieben, welche eine antiinflammatorische und analgetische Wirkung aufweisen.

Es wurde nun gefunden, daß die neuen Azepinderivate der allgemeinen Formel I

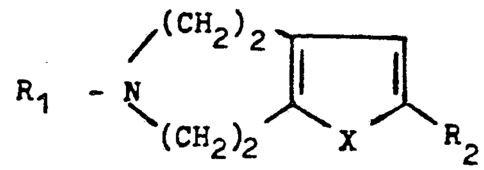

(I)

in der

$R_1$ ein Wasserstoffatom, eine Alkylgruppe mit 1 bis 12 Kohlenstoffatomen, eine Allylgruppe, eine gegebenenfalls im aromatischen Kern durch Hydroxygruppen und/oder Halogenatome mono- oder disubstituierte Aralkylgruppe mit 7 bis 9 Kohlenstoffatomen, eine gegebenenfalls durch Halogenatome und/oder Alkoxygruppen mit 1 bis 3 Kohlenstoffatomen mono- oder disubstituierte Benzoylgruppe, eine Alkoxycarbonylgruppe mit insgesamt 2 bis 4 Kohlenstoffatomen oder eine Aralkoxycarbonylgruppe mit insgesamt 8 bis 10 Kohlenstoffatomen,

$R_2$ ein Wasserstoffatom, eine Carboxygruppe oder eine Alkoxycarbonylgruppe mit insgesamt 2 bis 4 Kohlenstoffatomen und

X ein Sauerstoff- oder Schwefelatom oder eine Iminogruppe der Formel

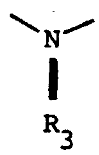

bedeuten, wobei $R_3$ ein Wasserstoffatom, eine gegebenenfalls durch eine Phenylgruppe substituierte Alkylgruppe mit 1 bis 3 Kohlenstoffatomen oder eine Phenylgruppe darstellt, bedeuten, und deren Salze mit anorganischen oder organischen Säuren oder auch Basen, wenn $R_2$ eine Carboxygruppe darstellt, insbesondere jedoch deren physiologisch verträgliche Salze, wertvolle pharmakologische Eigenschaften aufweisen; besonders hervorzuheben ist eine antithrombotische Wirkung und eine Wirkung auf den Intermediärstoffwechsel.

Gegenstand der vorliegenden Erfindung sind somit die obigen neuen Azepinderivate, deren Salze und diese Verbindungen enthaltende Arzneimittel sowie Verfahren zu ihrer Herstellung.

Für die bei der Definition der Reste $R_1$, $R_2$ und X eingangs erwähnten Bedeutungen kommt beispielsweise

für $R_1$ die Bedeutung des Wasserstoffatoms, der Methyl-, Äthyl-, Propyl-, Isopropyl-, Butyl-, Isobutyl-, Pentyl-, Hexyl-, Heptyl-, Octyl-, Nonyl-, Decyl-, Undecyl-, Dodecyl-, Allyl-, Benzyl-, 1-Phenyläthyl-, 1-Phenylpropyl-, 2-Phenyläthyl-, 3-Phenylpropyl-, Fluorbenzyl-, Chlorbenzyl-, Brombenzyl-, Dichlorbenzyl-, Dibrombenzyl-, Hydroxy-chlorbenzyl-, Hydroxy-brombenzyl-, 2-(Chlorphenyl)-äthyl-, Benzoyl-, Chlorbenzoyl-, Brombenzoyl-, Fluorbenzoyl-, Dichlorbenzoyl-, Methoxybenzoyl-, Propoxybenzoyl-, Chlor-methoxybenzoyl-, Brom-äthoxy- benzoyl-, Methoxycarbonyl-, Äthoxycarbonyl-, Propoxycarbonyl-, Isopropoxycarbonyl-, Benzyloxycarbonyl-, 1-Phenyläthyl- oxycarbonyl-, 2-Phenyläthyloxycarbonyl- oder 3-Phenylpropoxycarbonylgruppe,

für $R_2$ die des Wasserstoffatoms, der Carboxy-, Methoxycarbonyl-, Äthoxycarbonyl-, Propoxycarbonyl- oder Isopropoxycarbonylgruppe und

für X die eines Sauerstoff- oder Schwefelatoms, die einer Imino-, Methylimino-, Äthylimino-, Propylimino-, Isopropylimino-, Phenylimino-, Benzylimino-, 1-Phenyläthylimino-, 2-Phenyläthylimino-, 1-Phenylpropylimino- oder 3-Phenylpropyliminogruppe in Betracht.

Bevorzugte Verbindungen der obigen allgemeinen Formel (I) sind jedoch diejenigen, in denen

X ein Sauerstoff- oder Schwefelatom, eine Imino-, Methylimino-, Phenylimino- oder Benzyliminogruppe,

$R_1$ ein Wasserstoffatom, eine Alkylgruppe mit 1 bis 3 Kohlenstoffatomen, eine gegebenenfalls im Phenylkern durch Chlor- oder Bromatome mono- oder disubstituierte Benzylgruppe, eine im Phenylkern durch eine Hydroxygruppe und ein Chlor- oder Bromatom substituierte Benzylgruppe, eine gegebenenfalls durch ein Chlor- oder Bromatom substituierte Benzoylgruppe, eine Alkoxycarbonylgruppe mit insgesamt 2 bis 4 Kohlenstoffatomen, eine Allyl-, Dodecyl-, Chlor-methoxybenzoyl- oder Benzyloxycarbonylgruppe,

$R_2$ ein Wasserstoffatom, eine Carboxygruppe oder eine Alkoxycarbonylgruppe mit insgesamt 2 bis 4 Kohlenstoffatomen bedeuten, und deren Salze mit anorganischen oder organischen Säuren oder Basen, wenn $R_2$ eine Carboxygruppe darstellt, insbesondere deren physiologisch verträgliche Salze.

Besonders bevorzugte Verbindungen der obigen allgemeinen Formel I sind diejenigen, in denen

X ein Schwefelatom,

$R_1$ eine Dodecyl-, Benzyl-, Chlorbenzyl- oder Chlorbenzoylgruppe und

$R_2$ ein Wasserstoffatom oder die Carboxygruppe bedeuten, und deren physiologisch verträgliche Salze mit anorganischen oder organischen Säuren oder Basen.

Erfindungsgemäß erhält man die neuen Verbindungen nach folgenden Verfahren :

a) Cyclisierung einer gegebenenfalls im Reaktionsgemisch gebildeten Verbindung der allgemeinen Formel (II)

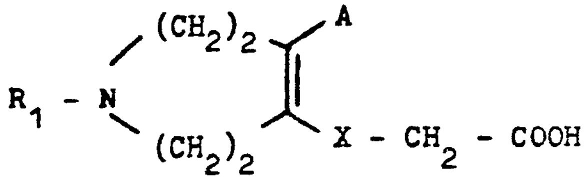

(II)

in der

$R_1$ und X wie eingangs definiert sind und

A die Formylgruppe oder deren Acetal darstellt, oder deren Ester und gegebenenfalls anschließende Hydrolyse und/oder Decarboxylierung.

Die Cyclisierung wird zweckmäßigerweise in einem Lösungsmittel wie Methanol, Äthanol, Äther, Dioxan, Pyridin, Triäthylamin oder deren Gemischen in Gegenwart einer Base wie Natriumcarbonat, Kaliumhydroxid, Natriumhydrid, Kalium-tert.butylat, Morpholin oder Diäthylamin oder einer wässrigen Base wie konzentrierte Kalilauge bei Temperaturen zwischen 0 und 100 °C, vorzugsweise jedoch bei Temperaturen zwischen 0 °C und der Siedetemperatur des Reaktionsgemisches, durchgeführt.

Die gegebenenfalls anschließende Hydrolyse wird zweckmäßigerweise in einem Lösungsmittel wie Wasser, Äthanol, Äthanol/Wasser, Dioxan oder Eisessig in Gegenwart einer Säure wie Salzsäure, Bromwasserstoffsäure oder Schwefelsäure oder in Gegenwart einer Base wie Natriumhydroxid oder Kaliumhydroxid bei Temperaturen zwischen 60 und 120 °C, vorzugsweise jedoch bei der Siedetemperatur des Reaktionsgemisches, durchgeführt.

Die gegebenenfalls anschließende Decarboxylierung wird vorzugsweise in Gegenwart einer Säure wie Salzsäure, Schwefelsäure, Bromwasserstoffsäure oder Phosphorsäure, welche gleichzeitig als Lösungsmittel dienen kann, oder durch Oxalsäure in einem Lösungsmittel wie Wasser, Äthanol/Wasser, Propanol, Eisessig/Wasser oder Dioxan/Wasser bei erhöhten Temperaturen, vorzugsweise bei der Siedetemperatur des Reaktionsgemisches, z. B. bei Temperaturen zwischen 80 und 100 °C, durchgeführt.

Eine als Ausgangsstoff verwendete Verbindung der allgemeinen Formel (II) wird hierbei vorzugsweise im Reaktionsgemisch durch Umsetzung eines entsprechenden Halogen-azepins mit einem entsprechenden Essigsäure-Derivat in einem Lösungsmittel wie Pyridin, Äthanol, Äther oder Dioxan und in Gegenwart einer Base wie Triäthylamin, Natriumcarbonat, Natriumhydrid, Natriummethylat oder Kalium-tert.butylat bei Temperaturen zwischen 0 und 50 °C hergestellt.

b) Zur Herstellung einer Verbindung der allgemeinen Formel (I), in der $R_2$ ein Wasserstoffatom darstellt :

Decarboxylierung einer gegebenenfalls im Reaktionsgemisch gebildeten Carbonsäure der allgemeinen Formel (III)

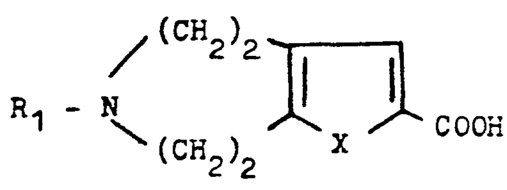

(III)

in der $R_1$ und X wie eingangs definiert sind.

Die Decarboxylierung wird vorzugsweise in Gegenwart einer Säure wie Salzsäure, Schwefelsäure, Bromwasserstoffsäure oder Phosphorsäure, welche gleichzeitig als Lösungsmittel dienen kann, oder auch Oxalsäure in einem Lösungsmittel wie Wasser, Äthanol/Wasser, Propanol, Dimethylsulfoxid, Eisessig/Wasser oder Dioxan/Wasser bei erhöhten Temperaturen, vorzugsweise bei der Siedetemperatur des Reaktionsgemisches, z. B. bei Temperaturen zwischen 80 und 100 °C, durchgeführt.

c) Zur Herstellung einer Verbindung der allgemeinen Formel (I), in der $R_1$ mit Ausnahme des Wasserstoffatoms wie eingangs definiert ist :

Umsetzung einer Verbindung der allgemeinen Formel (IV)

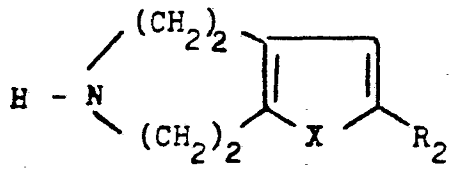

(IV)

in der $R_2$ und X wie eingangs definiert sind, mit einer Verbindung der allgemeinen Formel (V)

$$R_1{-}Z \qquad (V)$$

in der

$R_1$ mit Ausnahme des Wasserstoffatoms wie eingangs definiert ist und

Z eine nukleophile Austrittsgruppe, eine Hydroxygruppe oder zusammen mit einem Wasserstoffatom des benachbarten Kohlenstoffatoms ein Sauerstoffatom darstellt.

Als nukleophile Austrittsgruppe kommt beispielsweise ein Halogenatom wie das Chlor-, Brom- oder Jodatom, eine Sulfonyloxygruppe wie die Methansulfonyloxy-, Methoxy-sulfonyl-oxy- oder p-Toluolsulfonyloxygruppe oder zusätzlich bei der Acylierung auch eine Gruppe der Formel $-O-COR_1$ in Betracht.

Die Umsetzung wird zweckmäßigerweise in einem Lösungsmittel wie Äthanol, Isopropanol, Methylenchlorid, Chloroform, Acetonitril, Toluol, Dioxan, Dimethylformamid oder Decalin, gegebenenfalls in Gegenwart einer Base wie Triäthylamin, Pyridin oder Natriumkarbonat, gegebenenfalls in Gegenwart eines Hydrierungskatalysators wie Raney-Nickel oder Palladium/Kohle oder gegebenenfalls in Gegenwart eines Hydrierungskatalysators wie Raney-Nickel und bei einem Wasserstoffdruck von 1 bis 10 bar bei Temperaturen zwischen 0 und 250 °C, vorzugsweise jedoch bei Temperaturen zwischen 20 °C und der Siedetemperatur des eingesetzten Lösungsmittels durchgeführt.

Bedeutet Z eine nukleophile Austrittsgruppe, so wird die Umsetzung mit einem Alkylierungsmittel wie Äthyljodid, Benzylchlorid, Dimethylsulfat oder p-Toluolsulfonsäure-äthylester oder mit einem Acylierungsmittel wie Chlorameisensäureäthylester oder 2-Methoxy-5-chlor-benzoylchlorid vorzugsweise in einem Lösungsmittel und in Gegenwart einer Base bei Temperaturen zwischen 0 und 150 °C, vorzugsweise jedoch bei Temperaturen zwischen 20 und 80 °C, durchgeführt.

Bedeutet Z eine Hydroxygruppe, so wird die Umsetzung mit einem entsprechenden Alkohol, z. B. Methanol, Äthanol, Benzylalkohol oder o-Chlor-benzylalkohol, welcher gleichzeitig auch als Lösungsmittel dienen kann, vorzugsweise unter Stickstoff und in Gegenwart eines Hydrierungskatalysators bei Temperaturen zwischen 80 und 250 °C, vorzugsweise jedoch bei Temperaturen zwischen 100 °C und der Siedetemperatur des eingesetzten Alkohols, durchgeführt.

Bedeutet Z zusammen mit einem Wasserstoffatom des benachbarten Kohlenstoffatoms ein Sauerstoffatom, so wird die Umsetzung in einem Lösungsmittel und in Gegenwart von katalytisch angeregtem Wasserstoff bei Temperaturen zwischen 80 und 250 °C, vorzugsweise jedoch bei Temperaturen zwischen 100 und 200 °C, durchgeführt.

Erhält man erfindungsgemäß eine Verbindung der allgemeinen Formel (I), in der $R_1$ eine gegebenenfalls im aromatischen Kern durch Hydroxygruppen und/oder Halogenatome mono- oder disubstituierte Benzylgruppe und/oder X eine Benzyliminogruppe darstellt, so kann diese mittels katalytischer Hydrierung in eine entsprechende Verbindung der allgemeinen Formel (I), in der $R_1$ ein Wasserstoffatom und/oder X eine Iminogruppe darstellt, übergeführt werden und/oder eine Verbindung der allgemeinen Formel (I), in der $R_1$ eine gegebenenfalls im aromatischen Kern durch Hydroxygruppen und/oder Halogenatome mono- oder disubstituierte Benzylgruppe darstellt, so kann diese mittels Raney-Nickel in Gegenwart eines entsprechenden Alkohols in eine entsprechende Verbindung der allgemeinen Formel I, in der $R_1$ eine Alkylgruppe mit 1 bis 12 Kohlenstoffatomen darstellt, übergeführt werden oder eine erhaltende Verbindung der allgemeinen Formel (I), in der $R_1$ und/oder $R_2$ eine Alkoxycarbonylgruppe mit insgesamt 2 bis 4 Kohlenstoffatomen darstellt, mittels Hydrolyse und unter gleichzeitiger Decarboxylierung, falls $R_1$ eine Alkoxycarbonylgruppe mit insgesamt 2 bis 4 Kohlenstoffatomen darstellt, in eine entsprechende Verbindung der allgemeinen Formel (I), in der $R_1$ ein Wasserstoffatom und/oder $R_2$ eine Carboxygruppe darstellt, übergeführt werden.

Die nachträgliche katalytische Hydrierung wird vorzugsweise in einem Lösungsmittel wie Äthanol, Essigester oder Dioxan in Gegenwart eines Hydrierungskatalysators wie Palladium oder Platin und gegebenenfalls in Gegenwart einer Säure wie 1 N Salzsäure bei einem Wasserstoffdruck von 2 bis 10 bar und bei einer Temperatur zwischen 20 und 100 °C durchgeführt.

Die nachträgliche Umsetzung mit einem entsprechenden Alkohol in Gegenwart von Raney-Nickel wird vorzugsweise in dem entsprechenden Alkohol als Lösungsmittel bei erhöhten Temperaturen, z. B. bei Temperaturen zwischen 60 und 120 °C, durchgeführt.

Die nachträgliche Hydrolyse wird zweckmäßigerweise in einem Lösungsmittel wie Wasser, Äthanol, Äthanol/Wasser, Dioxan oder Eisessig in Gegenwart einer Säure wie Salzsäure, Bromwasserstoffsäure oder Schwefelsäure oder in Gegenwart einer Base wie Natriumhydroxid oder Kaliumhydroxid bei Temperaturen zwischen 60 und 120 °C, vorzugsweise jedoch bei der Siedetemperatur des Reaktionsgemisches, durchgeführt.

Außerdem kann ein erfindungsgemäß erhaltenes Isomerengemisch von Verbindungen der allgemeinen Formel (I) anschließend nach bekannten Methoden, z. B. durch Chromatographie an einem festen Träger wie Kieselgel oder durch fraktionierte Kristallisation, in seine Isomeren aufgetrennt werden.

Ferner lassen sich die erhaltenen Verbindungen der allgemeinen Formel (I) in ihre Salze, insbesondere in ihre physiologisch verträglichen Salze mit anorganischen oder organischen Säuren oder auch Basen, wenn $R_2$ eine Carboxygruppe darstellt, überführen. Als Säuren kommen hierbei beispielsweise Salzsäure, Bromwasserstoffsäure, Schwefelsäure, Phosphorsäure, Milchsäure, Oxalsäure, Zitronensäure, Weinsäure, Bernsteinsäure, Maleinsäure oder Fumarsäure und als Basen Natriumhydroxid, Kaliumhydroxid oder Cyclohexylamin in Betracht.

Die als Ausgangsstoffe verwendeten Verbindungen der allgemeinen Formeln (II) bis (V) sind teilweise literaturbekannt bzw. erhält man nach literaturbekanntenn Verfahren (siehe Beispiele I bis VII). So erhält man beispielsweise eine Verbindung der allgemeinen Formel (II) durch Vilsmeier-Reaktion mit einem entsprechenden Azepin. Die so erhaltene Chlor-formylverbindung kann anschließend mit einem entsprechenden Essigsäurederivat zu einer Verbindung der allgemeinen Formel (II) umgesetzt werden.

Eine als Ausgangsstoff verwendete Verbindung der allgemeinen Formel (III) oder (IV) erhält man durch Ringschluß eines entsprechenden Azepin-derivates mit einem entsprechenden Carbonsäure-Derivat und gegebenenfalls anschließender Entbenzylierung.

Wie bereits eingangs erwähnt, weisen die neuen Verbindungen wertvolle pharmakologische Eigenschaften auf, nämlich neben einer Hemmwirkung auf die Aggregationsneigung von Tumorzellen insbesondere eine antithrombotische Wirkung und eine auf den Intermediärstoffwechsel, insbesondere eine lipidsenkende Wirkung.

Beispielsweise wurden die Verbindungen

A = 6-Benzyl-5,6,7,8-tetrahydro-4H-thieno [2,3-d] azepin-2-carbonsäure-hydrochlorid

B = 6-Dodecyl-5,6,7,8-tetrahydro-4H-thieno [2,3-d] azepin-2-carbonsäure-natriumsalz und

C = 6-(2-Chlor-benzyl)-5,6,7,8-tetrahydro-4H-thieno [2,3-d] azepin-hydrochlorid

auf ihre biologischen Eigenschaften wie folgt untersucht :

1. Cholesterinsenkende Wirkung

a) Prüfung an der hyperlipämischen Ratte :

Literatur : P.E. Schurr et al. in Atherosclerosis Drug Discovery (1976), Herausgeber : C.E. Day ; Plenum, New York, Seite 215.

Junge männliche Ratten mit einem durchschnittlichen Gewicht von 100 g wurden durch viertägige Gabe einer Diät (bestehend aus 10 % Kokosfett, 1,5 % Cholesterin, 0,5 % Cholsäure, 0,2 % Cholinchlorid und 15 % Sucrose) hypercholesterinämisch gemacht. Unter Beibehaltung der Diät appliziert man an zwei aufeinanderfolgenden Tagen die zu untersuchende Substanz in Methylcellulose-Suspension per Schlundsonde. Anschließend wurden die Tiere über Nacht nüchtern gehalten und 24 Stunden nach der letzten Substanzapplikation wurde zur Serumgewinnung Blut entnommen.

Im Serum wurden Gesamtcholesterin (Boehringer Mannheim Testkombination 126 039) enzymatisch und die β-Lipoproteine nach Fällung mit $Ca^{++}$ und Heparin im Autoanalyser nephelometrisch bestimmt. Die Berechnung der prozentualen Senkung erfolgte gegen eine Kontrollgruppe.

| Substanz | Dosis mg/kg | Senkung in % gegenüber Kontrolle Serum-Gesamtcholesterin | Serum-β-Lipoproteine |
|---|---|---|---|
| B | 50 | − 21 | − 51 |

b) Prüfung an der normolipämischen Ratte :

Männlichen, normolipämischen Ratten mit einem durchschnittlichen Gewicht von 250-300 g wurden die zu untersuchenden Substanzen zweimal im Abstand von 20 Stunden per Schlundsonde appliziert. Bei Versuchsbeginn wurde den Tieren das Futter entzogen, Trinkwasser stand jedoch ad libitum zur Verfügung. Nach 28 und 44 Stunden wurden die Serum-Cholesterinspiegel (Boehringer Mannheim Testkombination 126 039) enzymatisch bestimmt. Die Berechnung der prozentualen Senkung erfolgte gegen eine Kontrollgruppe :

| Substanz | Dosis mg/kg | Senkung in % gegenüber Kontrolle Serum-Gesamtcholesterin |
|---|---|---|
| A | 1,0 | − 26 |
| B | 20,0 | − 20 |

2. Antithrombotische Wirkung :

a) Die Thrombocyten gesunder Versuchspersonen wurden für 10 Minuten bei 37 °C mit der zu untersuchenden Substanz inkubiert (Endkonzentration : $1 \times 10^{-4}$ Mol/1). Anschließend wurde mit Collagen in üblicher Weise die Aggregation im Born-Test ausgelöst. Kurz vor Aggregationsauslösung wurde der zu untersuchenden Probe eine kleine Menge prostacyclinhaltigen Überstandes zugesetzt, der aus in TRIS-Puffer eingelegten Aortenringen gewonnen wurde. Die Menge des prostacyclinhaltigen Überstandes wurde so gewählt, daß sie allein keine oder nur eine sehr geringe Hemmwirkung entfaltete. Die mit der Substanz inkubierte Probe reagierte mit $PGI_2$ mit einer mehr als additiven Aggregationshemmung. Dies zeigt die nachfolgende Tabelle :

| | n | mm Kurvenhöhe (Born-Test) | % Hemmung der Thrombocyten-aggregation |
|---|---|---|---|
| Kontrolle | 4 | 129 | |
| PRP+ + Substanz C | 4 | 101 | 20,7 |
| PRP + $PGI_2$ | 4 | 90 | 30 |
| PRP + Substanz C + $PGI_2$ | 4 | 38 | 69 |

+ PRP = plättchenreiches Plasma

b) Nachweis einer adenosinpotenzierenden Wirkung im Borntest :

Technisch wurde in gleicher Weise wie unter Punkt 2a beschrieben vorgegangen. Die Adenosinmenge wurde so gewählt, daß sie allein nur eine geringe Hemmwirkung entfaltet. Die nachfolgende Tabelle zeigt, daß die Substanz plus Adenosin eine mehr als additive Hemmwirkung entfaltet.

| | n | mm Kurvenhöhe im Born-Test | % Aggregations-Hemmung |
|---|---|---|---|
| Kontrolle | 4 | 114 | |
| PRP+ + Substanz C ($1 \times 10^{-4}$) | 4 | 75 | 33 |
| PRP+ + Adenosin | 4 | 91 | 20 |
| PRP+ + Substanz C + Adenosin | 4 | 44 | 61 |

+ PRP = plättchenreiches Plasma

c) Die prostacyclinverstärkende Wirkung kann auch im Tierversuch nach oraler Substanzapplikation nachgewiesen werden : Ratten im Gewicht von 450 g wurde die Substanz in der Dosierung von 20 mg/kg oral appliziert, nach einer Stunde wurde Blut entnommen und der Born-Test wie oben beschrieben durchgeführt. Die nachfolgende Tabelle zeigt, daß auch hier ein den in vitro Versuchen entsprechender, überadditiver Effekt zusammen mit $PGI_2$ auftritt (Mittelwert aus 3 Versuchskollektiven à 4 Tieren) :

| |
|---|
| Mit 20 mg/kg p. o. Substanz C behandelte Tiere : 4 % Aggr.-Hemmung |
| Plasma von Kontrolltieren plus $PGI_2$ : 20 % Aggr.-Hemmung |
| Mit Substanz C behandelte Tiere + $PGI_2$ : 100 % Aggr.-Hemmung |

3. Akute Toxizität :

Die Verbindungen A, B und C zeigen bei den höchsten applizierten Dosen (50 mg/kg p. o.) keinerlei toxische Nebenwirkungen.

Aufgrund ihrer pharmakologischen Eigenschaften eignen sich die erfindungsgemäß hergestellten Verbindungen welche cholesterinsenkende Eigenschaften aufweisen, zur Behandlung von Hyperlipidämien, insbesondere des Typs IIA, IIB und IV, und dadurch bedingten atherosklerotischen Veränderungen des Gefäßsystems und diejenigen, welche antithrombotische Eigenschaften aufweisen, zur Prophylaxe

thromboembolischer Erkrankungen wie Coronarinfarkt, Cerebralinfarkt, sogn. transient ischaemic attacks, Amaurosis fugax sowie zur Prophylaxe der Arteriosklerose und der Metastasenbildung.

Hierzu lassen sich diese, gegebenenfalls in Kombination mit anderen Wirksubstanzen, in die üblichen pharmazeutischen Zubereitungsformen wie Dragées, Tabletten, Kapseln, Suppositorien, Ampullen, Tropfen oder Suspensionen einarbeiten.

Zur Behandlung von Hyperlipidämien beträgt die Tagesdosis am Erwachsenen 10 bis 200 mg, vorzugsweise 15 bis 150 mg, und bei der

Behandlung von thrombo-embolischen Erkrankungen sowie der Prophylaxe der Arteriosklerose und der Metastasenbildung 500 bis 1 000 mg, vorzugsweise 600 bis 900 mg, verteilt auf 2 bis 4 Einzeldosen.

Die nachfolgenden Beispiele sollen die Erfindung näher erläutern :

Herstellung der Ausgangsprodukte :

## Beispiel I

1-Benzyl-4-chlor-5-formyl-2,3,6,7-tetrahydro-1H-azepinhydrochlorid

Zu dem aus 460 g (3 Mol) Phosphoroxychlorid und 292 g (4 Mol) Dimethylformamid hergestellten Vilsmeier-Komplex werden bei Raumtemperatur 400 ml Methylenchlorid und anschließend 240 g (1 Mol) 1-Benzyl-hexahydro-azepinon-(4)-hydrochlorid in 2 Portionen innerhalb einer Stunde zugegeben, wobei die Temperatur im Reaktionsgemisch auf etwa 20 °C gehalten wird. Danach wird noch 5 Stunden nachgerührt. Nach Stehen über Nacht wird auf 1 kg Eis gegossen und nach 3-stündigem Stehen die Methylenchlorid-Phase abgetrennt. Die wässrige Phase wird mehrmals mit Chloroform ausgeschüttelt. Die vereinigten organischen Extrakte werden über Natriumsulfat getrocknet und am Rotationsverdampfer eingedampft. Der halbfeste Rückstand wird mit 200 ml Isopropanol verrieben, abgesaugt und mit wenig eiskaltem Isopropanol nachgewaschen.

Ausbeute : 85,3 g (30 % der Theorie).

Schmelzpunkt : 215-216 °C

| | | | | |
|---|---|---|---|---|
| Ber. : | C 58,76 | H 5,99 | N 4,90 | Cl 24,77 |
| Gef. : | C 58,60 | H 6,06 | N 4,81 | Cl 24,50 |

## Beispiel II

4-Chlor-5-formyl-2,3,6,7-tetrahydro-1H-1-azepincarbonsäure-äthylester und 4-Chlor-3-formyl-2,5,6,7-tetrahydro-1H-1-azepincarbonsäure-äthylester

Zu dem aus 113,8 g (1,5 Mol) Dimethylformamid und 120 g (0,78 Mol) Phosphoroxychlorid hergestellten Vilsmeier-Komplex werden bei 40 °C 111 g (0,6 Mol) Hexahydro-azepinon-(4)-1-carbonsäure-äthylester tropfenweise unter Rühren gegeben, wobei die Temperatur der Reaktionsmischung unterhalb 50 °C gehalten wird. Nach der Zugabe wird noch 2 Stunden nachgerührt. Danach wird in 1,4 Liter Eiswasser eingerührt und die wässrige Lösung 2 mal mit Äther extrahiert. Die vereinigten Ätherextrakte werden 2 mal mit Wasser gewaschen und über Natriumsulfat getrocknet. Nach Abdestillieren des Lösungsmittels am Rotationsverdampfer hinterbleibt ein gelbes Öl (1 : 1-Gemisch der beiden Isomeren), das ohne Reinigung weiter umgesetzt wird.

Ausbeute : 93 g (67 % der Theorie).

## Beispiel III

4-Chlor-5-formyl-2,3,6,7-tetrahydro-1H-1-azepincarbonsäure-benzylester und 4-Chlor-3-formyl-2,5,6,7-tetrahydro-1H-1-azepincarbonsäure-benzylester

Das aus 18,9 g (0,26 Mol) Dimethylformamid und 20 g (0,13 Mol) Phosphoroxychlorid hergestellte Vilsmeier-Reagens wird bei 40-45 °C tropfenweise unter Rühren mit 24,3 g (0,1 Mol) Hexahydro-azepinon-(4)-1-carbonsäure-benzylester versetzt, wobei die Temperatur im Reaktionsgemisch unterhalb 50 °C gehalten wird. Nach der Zugabe wird noch 2 Stunden nachgerührt. Danach wird in 250 ml Eiswasser eingerührt und die wässrige Lösung 3 mal mit Äther extrahiert. Die vereinigten Ätherextrakte werden 2 mal mit Wasser gewaschen und über Natriumsulfat getrocknet. Nach Abdestillieren des Lösungsmittels hinterbleibt ein gelbliches Öl (1 : 1-Gemisch der beiden Isomeren), das ohne Reinigung weiter umgesetzt wird.

Ausbeute : 17,1 g (60 % der Theorie).

## Beispiel IV

4-Chlor-5-formyl-2,3,6,7-tetrahydro-1H-1-azepincarbonsäure-äthylester

In eine Lösung von 7,5 g (0,03 Mol) 1-Benzyl-4-chlor-5-formyl-2,3,6,7-tetrahydro-1H-azepin in 50 ml

trockenem Methylenchlorid werden unter Rühren bei Raumtemperatur innerhalb von 30 Minuten 3,6 g (0,033 Mol) Chlorameisensäure-äthylester, gelöst in 15 ml trockenem Methylenchlorid, getropft, wobei die Temperatur des Reaktionsgemisches von 22 °C auf 27 °C ansteigt. Nach beendeter Zugabe wird noch 3 Stunden bei Raumtemperatur weitergerührt. Danach wird das Methylenchlorid am Rotationsverdampfer abdestilliert und der Rückstand am Hochvakuum destilliert.

Ausbeute : 6,5 g (93,8 % der Theorie),
Kp. 0,26 mbar : 116-118 °C.
Ber. : C 51,84 H 6,09 N 6,05 Cl 15,30
Gef. : C 52,08 H 6,05 N 5,84 Cl 15,27

Beispiel V

4-Chlor-5-formyl-2,3,6,7-tetrahydro-1H-1-azepincarbonsäure-benzylester

Hergestellt aus 40 g (0,16 Mol) 1-Benzyl-4-chlor-5-formyl-2,3,6,7-tetrahydro-1H-azepin und 34,2 g (0,20 Mol) Chlorameisensäurebenzylester analog Beispiel IV.

Ausbeute : 95 % der Theorie,
Ber. : Molpeak m/e = 293/295 (1 Cl)
Gef. : Molpeak m/e = 293/295 (1 Cl)

Beispiel VI

4-Chlor-5-formyl-2,3,6,7-tetrahydro-1H-azepin-hydrobromid

10,5 g (0,036 Mol) 4-Chlor-5-formyl-2,3,6,7-tetrahydro-1H-1-azepincarbonsäure-benzylester werden unter Eiswasserkühlung und Rühren mit 100 ml einer 40 %igen Lösung von Bromwasserstoft in Eisessig versetzt. Anschließend wird 3 Stunden bei Raumtemperatur nachgerührt. Danach wird am Rotationsverdampfer eingeengt und der Rückstand mit Äther verrieben. Nach mehrmaligem Abdekantieren des Äthers wird abgesaugt und zweimal mit absolutem Äther gewaschen.

Ausbeute : 8,0 g (93 % der Theorie),
Schmelzpunkt : 204-206 °C.

Beispiel VII

1-(2-Chlor-benzyl)-4-chlor-5-formyl-2,3,6,7-tetrahydro-1H-azepin-hydrochlorid

Hergestellt analog Beispiel I aus 1-(2-Chlor-benzyl)-hexahydro-azepinon-(4)-hydrochlorid durch Vilsmeier-Reaktion mit Dimethylformamid/Phosphoroxychlorid.

Ausbeute : 42 % der Theorie,
Schmelzpunkt : 202-204 °C
Ber. : C 52,44 H 5,03 Cl 33,17 N 4,37
Gef. : C 52,55 H 5,08 Cl 32,65 N 4,50

Herstellung der Endprodukte

Beispiel 1

6-Benzyl-5,6,7,8-tetrahydro-4H-thieno [2,3-d] azepin-2-carbonsäure-äthylester

Eine Suspension von 85,3 g (0,298 Mol) 1-Benzyl-4-chlor-5-formyl-2,3,6,7-tetrahydro-1H-azepin-hydrochlorid in 350 ml Pyridin wird bei Raumtemperatur mit 43 g (0,358 Mol) Thioglykolsäure-äthylester versetzt. Innerhalb von 1,5 Stunden werden unter Rühren 75 g (0,75 Mol) absolutes Triäthylamin zugetropft, wobei allmähliche Lösung eintritt. Durch Kühlen mit Eiswasser wird die Temperatur unterhalb 35 °C gehalten. Danach wird 3 Stunden bei Raumtemperatur nachgerührt und über Nacht stehengelassen. Anschließend werden unter starkem Rühren und Eiskühlung 8 ml einer 48 %igen wässrigen Kaliumhydroxyd-Lösung langsam zugetropft und 2 Stunden unter Eiskühlung nachgerührt. Das Reaktionsgemisch wird nun in Eiswasser gegossen, wobei das zunächst ausfallende Öl nach einiger Zeit durchkristallisiert. Die Kristalle werden abgesaugt, mehrmals mit Wasser gewaschen und aus wenig Isopropylalkohol umkristallisiert.

Ausbeute : 68 g (72 % der Theorie),
Schmelzpunkt : 65-66 °C
Ber. : C 68,54 H 6,71 N 4,44 S 10,17
Gef. : C 68,76 H 6,92 N 4,42 S 10,05

Beispiel 2

5,6,7,8-Tetrahydro-4H-thieno [2,3-d] azepin-2-carbonsäure-äthylester-hydrochlorid

15,4 g (0,049 Mol) 6-Benzyl-5,6,7,8-tetrahydro-4H-thieno-[2,3-d] azepin-2-carbonsäure-äthylester werden unter Zusatz von 50 ml In Salzsäure in 200 ml Äthylalkohol gelöst. Nach Zugabe von 5 g Palladiumoxyd wird im Autoklaven 3 Stunden bei 80 °C und einem Wasserstoffdruck von 5 bar hydriert. Nach Abkühlen des Reaktionsgemisches wird der Katalysator abfiltriert, das Filtrat eingedampft und der Rückstand mit Aceton verrieben. Das Produkt wird abgesaugt und mehrmals mit Aceton nachgewaschen.

Ausbeute : 12,8 g (91 % der Theorie),
Schmelzpunkt : 159-160 °C.

| | | | | | |
|---|---|---|---|---|---|
| Ber. : | C 50,47 | H 6,17 | N 5,35 | Cl 13,54 | S 12,25 |
| Gef. : | C 50,63 | H 6,40 | N 5,23 | Cl 13,56 | S 12,15 |

Beispiel 3

6-Äthyl-5,6,7,8-tetrahydro-4H-thieno [2,3-d] azepin-2-carbonsäure-äthylester-hydrochlorid

6,9 g (0,030 6 Mol) 5,6,7,8-Tetrahydro-4H-thieno [2,3-d] azepin-2-carbonsäure-äthylester werden in 60 ml Chloroform und 4,6 g = 6,4 ml (0,046 Mol) Triäthylamin gelöst und zum Sieden erhitzt. Unter Rühren werden 5,5 g = 2,85 ml (0,035 Mol) Äthyljodid, gelöst in 10 ml Chloroform, langsam zugetropft und 3 Stunden weitererhitzt. Nach dieser Zeit werden nochmals 1,3 ml Triäthylamin und 0,57 ml Äthyljodid zugegeben. Nach weiterem 2-stündigem Kochen werden dieselben Mengen ein zweites Mal zugesetzt. Nach insgesamt 7-stündigem Erhitzen ist die Reaktion beendet. Man läßt die Reaktionsmischung abkühlen, extrahiert 3 mal mit Wasser und trocknet über Natriumsulfat. Nach Einengen der Chloroformphase am Rotationsverdampfer hinterbleiben 8 g eines braunen Öls. Durch Lösen in Aceton und Versetzen mit ätherischer Salzsäure wird das Hydrochlorid gefällt, das nach Absaugen aus Isopropylalkohol umkristallisiert wird.

Ausbeute : 5,6 g (63,1 % der Theorie),
Schmelzpunkt : 198-199 °C.

| | | | | | |
|---|---|---|---|---|---|
| Ber. : | C 53,88 | H 6,96 | N 4,83 | Cl 12,23 | S 11,06 |
| Gef. : | C 53,90 | H 7,06 | N 4,86 | Cl 12,40 | S 11,10 |

Beispiel 4

6-Äthyl-5,6,7,8-tetrahydro-4H-thieno [2,3-d] azepin-2-carbonsäure-äthylester

3,15 g (0,01 Mol) 6-Benzyl-5,6,7,8-tetrahydro-4H-thieno [2,3-d] azepin-2-carbonsäure-äthylester werden mit derselben Menge Raney-Nickel in 50 ml Äthylalkohol 6 Stunden am Rückfluß erhitzt. Danach wird vom Katalysator abgesaugt und das Filtrat eingedampft.

Ausbeute : 2,1 g (66,6 % der Theorie), gelbes Öl.
Ber. : Molpeak m/e = 253
Gef. : Molpeak m/e = 253

Beispiel 5

5,6,7,8-Tetrahydro-4H-thieno [2,3-d] azepin-2,6-dicarbonsäurediäthylester und 5,6,7,8-Tetrahydro-4H-thieno [3,2-c] azepin-2,5-dicarbonsäurediäthylester (wird nicht beansprucht)

92,5 g (0,4 Mol) eines Isomerengemisches bestehend aus 4-Chlor-5-formyl-2,3,6,7-tetrahydro-1H-1-azepincarbonsäure-äthylester und 4-Chlor-3-formyl-2,5,6,7-tetrahydro-1H-1-azepincarbonsäure-äthylester werden in 400 ml Pyridin gelöst. Nach Zugabe von 57,5 g = 52,5 ml (0,478 Mol) Thioglykolsäureäthylester werden unter Eiskühlung und starkem Rühren 60 g = 82,5 ml (0,594 Mol) Triäthylamin innerhalb von 1,5 Stunden zugetropft, wobei die Temperatur unterhalb von 25 °C gehalten werden soll. Man rührt anschließend noch 4 Stunden weiter und läßt dann über Nacht stehen. Anderntags wird eine Lösung von 33,5 g (0,6 Mol) Kaliumhydroxid in 40 ml Wasser unter starkem Rühren und Kühlen langsam zugetropft. Dabei soll die Temperatur 10 °C nicht übersteigen. Danach wird noch eine Stunde nachgerührt. Anschließend wird die Reaktionsmischung auf Eiswasser gegossen und mehrmals mit Äther unter Zusatz von etwas Essigsäureäthylester extrahiert. Die vereinigten Extrakte werden 3 mal mit Wasser geschüttelt und über Natriumsulfat getrocknet. Nach Abdestillieren des Lösungsmittels im Vakuum erhält man ein gelbes Öl, das an einer Kiesegelsäule (1,5 l ; Toluol/Aceton = 19 : 1) chromatographisch gereinigt wird.

Ausbeute : 71,5 g (60 % der Theorie).

| | | | | |
|---|---|---|---|---|
| Ber. : | C 56,55 | H 6,44 | N 4,71 | S 10,78 |
| Gef. : | C 56,50 | H 6,52 | N 4,97 | S 10,82 |

Analog wurde folgendes Gemisch hergestellt :

6-Benzyloxycarbonyl-5,6,7,8-tetrahydro-4H-thieno [2,3-d] azepin-2-carbonsäureäthylester und 5-Benzyloxycarbonyl-5,6,7,8-tetrahydro-4H-thieno [3,2-c] azepin-2-carbonsäureäthylester

Ausbeute : 39,5 % der Theorie, Öl (2 : 1-Gemisch).
Ber. : C 63,49 H 5,89 N 3,90 S 8,92
Gef. : C 63,68 H 5,87 N 3,83 S 9,03

## Beispiel 6

6-Carbäthoxy-5,6,7,8-tetrahydro-4H-thieno [2,3-d] azepin-2-carbonsäure und 5-Carbäthoxy-5,6,7,8-tetrahydro-4H-thieno [3,2-c] azepin-2-carbonsäure (wird nicht beansprucht)

10 g (0,034 Mol) eines Isomerengemisches bestehend aus 5,6,7,8-Tetrahydro-4-thieno [2,3-d] azepin-2,6-dicarbonsäurediäthylester und 5,6,7,8-Tetrahydro-4H-thieno [3,2-c] azepin-2,5-dicarbonsäure-diäthylester werden in einer Lösung von 2,8 g (0,05 Mol) Kaliumhydroxid in 150 ml absolutem Äthylalkohol 4 Stunden am Rückfluß gekocht. Danach wird im Vakuum eingedampft, der Rückstand in Wasser aufgenommen und 3 mal mit Äther extrahiert. Die Ätherextrakte werden verworfen und die wässrige Phase mit konzentrierter Salzsäure angesäuert. Es wird mehrfach mit Äther ausgeschüttelt, die vereinigten Ätherphasen mit Wasser gewaschen und über Natriumsulfat getrocknet. Nach Abdestillieren des Äthers hinterbleiben 9,2 g eines gelblichen Öls.

Zur Isomerentrennung wird das Gemisch auf einer Kieselgelsäule (1,3 l, Toluol/Eisessig = 9 : 1) chromatographiert. Die Trennung wird dünnschichtchromatographisch verfolgt. Nach Vereinigung der Fraktionen, die nur ein Isomeres enthalten, wird das Lösungsmittel abdestilliert.

Ausbeute des [2,3-d] Isomeren : 1,05 g (11,6 % der Theorie),
Schmelzpunkt : 170-171 °C
Ber. : C 53,52 H 5,61 N 5,20
Gef. : C 53,00 H 5,63 N 5,27
Ausbeute des [3,2-c] Isomeren : 3,05 g (33,9 % der Theorie),
Schmelzpunkt : 135-137 °C.
Ber. : C 53,52 H 5,61 N 5,20 S 11,90
Gef. : C 53,60 H 5,74 N 5,28 S 12,20

## Beispiel 7

5,6,7,8-Tetrahydro-4H-thieno [2,3-d] azepin-2-carbonsäure-äthylester und 5,6,7,8-Tetrahydro-4H-thieno [3,2-c] azepin-2-carbonsäure-äthylester (wird nicht beansprucht)

82,5 g (0,275 Mol) eines Isomerengemisches bestehend aus 5,6,7,8-Tetrahydro-4H-thieno [2,3-d] azepin-2,6-dicarbonsäurediäthylester und 5,6,7,8-Tetrahydro-4H-thieno [3,2-c] azepin-2,5-dicarbonsäure-diäthylester werden in der Siedehitze unter Rühren in eine Lösung von 154 g (2,75 Mol) Kaliumhydroxyd in 1,3 l absolutem Äthylalkohol getropft und 1 Stunde unter Rückfluß weitererhitzt. Danach wird das gebildete Äthanol langsam abdestilliert. Zur quantitativen Entfernung des Alkohols wird gegen Ende der Destillation Vakuum angelegt. Der trockene Rückstand, bestehend aus einem Gemisch der Kaliumsalze der 5,6,7,8-Tetrahydro-4H-thieno [2,3-d] azepin-2-carbonsäure und der 5,6,7,8-Tetrahydro-4H-thieno [3,2-c] azepin-2-carbonsäure wird in 1,3 l absolutem Alkohol suspendiert und zunächst bei Raumtemperatur 3 und bei Rückflußtemperatur 1,5 Stunden Chlorwasserstoff eingeleitet. Dieses Vorgehen wird nach 48-stündigem Stehen wiederholt. Danach wird abgekühlt, vom ausgefallenen Kaliumchlorid abgesaugt und das Filtrat im Vakuum eingedampft. Der Rückstand wird in Wasser gelöst und 2 mal mit Äther extrahiert. Die wässrige Phase wird mit Natriumcarbonat alkalisch gestellt und 3 mal mit Chloroform extrahiert. Nach 2-maligem Waschen der vereinigten Chloroform-Extrakte mit Wasser und Trocknen über Natriumsulfat wird im Vakuum eingedampft.

Ausbeute an Isomerengemisch : 22,6 g (36 % der Theorie).

Zur Isomerentrennung werden 10 g des Isomerengemisches auf einer Kieselgelsäule (1,9 l ; Chloroform/Äthylalkohol/-Ammoniak = 9/1, 3/0,07) chromatographiert. Die Trennung wird dünnschichtchromatographisch verfolgt. Nach Vereinigung der Fraktionen, die nur ein Isomeres enthalten, wird das Lösungsmittel abdestilliert.

Ausbeute des [2,3-d] Isomeren : 3,3 g (11,9 % der Theorie)
Ber. : C 58,64 H 6,71 N 6,22 S 14,20
Gef. : C 58,32 H 6,92 N 6,39 S 14,06
Ausbeute des [3,2-c] Isomeren : 4,7 g (16,8 % der Theorie) (wird nicht beansprucht)
Ber. : Molpeak m/e = 225
Gef. : Molpeak m/e = 225
Schmelzpunkt des Hydrochlorids : 252 °C (zers.)

Ber. : C 50,70 H 6,17 N 5,28 Cl 13,35 S 12,20
Gef. : C 50,47 H 6,16 N 5,35 Cl 13,54 S 12,25

Beispiel 8

6-Benzyl-5,6,7,8-tetrahydro-4H-thieno [2,3-d] azepin-2-carbonsäure-hydrochlorid

21,5 g (0,068 Mol) 6-Benzyl-5,6,7,8-tetrahydro-4H-thieno [2,3-d] azepin-2-carbonsäure-äthylester werden unter Zusatz von 50 ml Wasser in 100 ml konz. Salzsäure auf dem Dampfbad erhitzt, wobei die Substanz allmählich in Lösung geht. Nach 1,5 Stunden werden nochmals 20 ml konz. Salzsäure zugesetzt und weitere 30 Minuten erhitzt. Man läßt über Nacht stehen, saugt das ausgefallene Produkt ab und kristallisiert aus Äthanol um.

Ausbeute : 8,1 g (37 % der Theorie),
Schmelzpunkt : 260-262 °C (Zers.).
Ber. : C 59,34 H 5,60 N 4,33 Cl 10,95 S 9,90
Gef. : C 59,40 H 5,76 N 4,25 Cl 11,10 S 10,00

Analog Beispiel 1, wobei die entsprechende 4-Chlor-5-formyl-Verbindung als Zwischenprodukt nicht immer isoliert wurde, und den Beispielen 3, 7 und 8 wurden folgende Verbindungen hergestellt :

6-Äthyl-5,6,7,8-tetrahydro-4H-thieno [2,3-d] azepin-2-carbonsäure-äthylester-hydrochlorid

Ausbeute : 22 % der Theorie,
Schmelzpunkt : 198-199 °C

6-Äthyl-5,6,7,8-tetrahydro-4H-thieno [2,3-d] azepin-2-carbonsäure-hydrochlorid

Ausbeute : 51,3 % der Theorie,
Schmelzpunkt : 299 °C (Zers.).
Ber. : C 50,47 H 6,16 N 5,35 Cl 13,54 S 12,25
Gef. : C 50,70 H 6,26 N 5,34 Cl 13,68 S 12,18

6-Allyl-5,6,7,8-tetrahydro-4H-thieno [2,3-d] azepin-2-carbonsäure-äthylester-hydrochlorid

Ausbeute : 31 % der Theorie,
Schmelzpunkt : 196-197 °C
Ber. : C 55,71 H 6,68 N 4,64 Cl 11,74
Gef. : C 55,70 H 6,75 N 4,63 Cl 11,75

6-Allyl-5,6,7,8-tetrahydro-4H-thieno [2,3-d] azepin-2-carbonsäure-hydrochlorid

Ausbeute : 44,4 % der Theorie,
Schmelzpunkt : 276 °C (Zers.).
Ber. : C 52,65 H 5,89 N 5,12 Cl 12,95 S 11,71
Gef. : C 52,70 H 5,99 N 5,06 Cl 13,00 S 11,68

6-Allyl-5,6,7,8-tetrahydro-4H-thieno [2,3-d] azepin-2-carbonsäure-äthylester

Ausbeute : 81 % der Theorie, braunes Öl
Ber. : C 63,36 H 7,22 N 5,28
Gef. : C 63,19 H 7,34 N 5,51

6-(2-Chlorbenzyl)-5,6,7,8-tetrahydro-4H-thienc [2,3-d] azepin-2-carbonsäure-äthylester-hydrochlorid

Ausbeute : 76,6 % der Theorie,
Schmelzpunkt : 191-192 °C
Ber. : C 55,96 H 5,48 N 3,62 Cl 18,35 S 8,30
Gef. : C 56,20 H 5,46 N 3,66 Cl 18,55 S 8,45

6-(2-Chlorbenzyl)-5,6,7-8-tetrahydro-4H-thieno [2,3-d] azepin-2-carbonsäure-hydrochlorid

Ausbeute : 71,9 % der Theorie,
Schmelzpunkt : 247-248 °C
Ber. : C 53,64 H 4,78 N 3,91 Cl 19,79 S 8,95
Gef. : C 53,60 H 4,74 N 3,90 Cl 19,65 S 8,76

6-(4-Chlorbenzyl)-5,6,7,8-tetrahydro-4H-thieno [2,3-d] azepin-2-carbonsäure-äthylester

Ausbeute : 79 % der Theorie,
Schmelzpunkt : 89-90 °C
Ber. : C 61,79 H 5,76 N 4,00 Cl 10,13 S 9,16
Gef. : C 61,70 H 5,87 N 3,98 Cl 10,30 S 9,15

6-(4-Chlorbenzyl-5,6,7,8-tetrahydro-4H-thieno [2,3-d] azepin-2-carbonsäure-hydrochlorid

Ausbeute : 84,6 % der Theorie,
Schmelzpunkt : 253-254 °C (Zers.).
Ber. : C 53,64 H 4,78 N 3,91 Cl 19,79 S 8,95
Gef. : C 53,80 H 4,83 N 3,84 Cl 19,92 S 8,98

6-Dodecyl-5,6,7,8-tetrahydro-4H-thieno [2,3-d] azepin-2-carbonsäure-Natriumsalz

Ausbeute : 36,2 % der Theorie,
Schmelzpunkt : Ab 305 °C (Zers.).
Ber. : C 65,08 H 8,84 N 3,61 S 8,27
Gef. : C 65,30 H 8,91 N 3,45 S 8,22

6-(5-Chlor-2-methoxy-benzoyl)-5,6,7,8-tetrahydro-4H-thieno [2,3-d] azepin-2-carbonsäure-äthylester

Ausbeute : 21,6 % der Theorie,
Schmelzpunkt : 192-193 °C
Ber. : C 57,94 H 5,12 N 3,56 Cl 9,00 S 8,14
Gef. : C 58,30 H 5,18 N 3,61 Cl 9,11 S 8,16

6-(5-Chlor-2-methoxy-benzoyl)-5,6,7,8-tetrahydro-4H-thieno [2,3-d] azepin-2-carbonsäure

Ausbeute : 81,1 % der Theorie,
Schmelzpunkt : 234-236 °C
Ber. : C 55,82 H 4,41 N 3,83 S 8,76
Gef. : C 56,00 H 4,58 N 3,90 S 8,82

5,6,7,8-Tetrahydro-6-propyl-4H-thieno [2,3-d] azepin-2-carbonsäure-äthylester

Ausbeute : 93 % der Theorie, Öl
Schmelzpunkt des Hydrochlorids : 222-224 °C
Ber. : C 55,34 H 7,30 N 4,61 Cl 11,67 S 10,55
Gef. : C 55,52 H 7,17 N 4,48 Cl 11,83 S 10,82

5,6,7,8-Tetrahydro-6-isopropyl-4H-thieno [2,3-d] azepin-2-carbonsäure-äthylester

Ausbeute : 97 % der Theorie, Öl
Schmelzpunkt des Hydrochlorids : 225-227 °C
Ber. : C 55,34 H 7,30 N 4,61 Cl 11,67 S 10,55
Gef. : C 55,35 H 7,24 N 4,59 Cl 11,77 S 10,77

5,6,7,8-Tetrahydro-6-methyl-4H-thieno [2,3-d] azepin-2-carbonsäure-äthylester

Ausbeute : 42 % der Theorie, Öl
Schmelzpunkt des Hydrochlorids : 237-239 °C
Ber. : C 52,26 H 6,58 N 5,08 Cl 12,86 S 11,62
Gef. : C 52,36 H 6,51 N 5,21 Cl 12,90 S 11,90

## Beispiel 9

6-Benzyl-5,6,7,8-tetrahydro-4H-thieno [2,3-d] azepin-hydrochlorid

3,1 g (0,096 Mol) 6-Benzyl-5,6,7,8-tetrahydro-4H-thieno [2,3-d] azepin-2-carbonsäure-hydrochlorid werden in 30 ml konzentrierter Salzsäure 48 Stunden am Rückfluß erhitzt. Nach dem Erkalten wird im Vakuum eingeengt, mit Wasser versetzt, mit 6 N Natronlauge alkalisch gestellt und mehrmals mit Äther ausgeschüttelt. Die Ätherphasen werden nochmals mit Wasser gewaschen, über Natriumsulfat getrocknet und im Vakuum eingedampft. Nach nochmaligem Eindampfen mit Benzol hinterbleibt ein braunes Öl. Es wird in Isopropylalkohol gelöst, mit isopropylalkoholischer Salzsäure das Hydrochlorid gefällt und aus Isopropylalkohol umkristallisiert.

Ausbeute : 1,8 g (67 % der Theorie),
Schmelzpunkt : 237-239 °C
Ber. : C 64,38 H 6,48 N 5,01 Cl 12,67 S 11,46
Gef. : C 64,20 H 6,60 N 4,88 Cl 12,70 S 11,70

Beispiel 10

6-(2-Chlorbenzyl)-5,6,7,8-tetrahydro-4H-thieno [2,3-d] azepin-hydrochlorid

6,9 g (0,045 Mol) 5,6,7,8-Tetrahydro-4H-thieno [2,3-d] azepin werden mit 5,6 g = 8,4 ml (0,055 Mol) absolutem Triäthylamin und 7,97 g = 6,2 ml (0,049 5 Mol) o-Chlor-benzylchlorid in 120 ml Toluol 10 Stunden am Rückfluß erhitzt, wobei nach 5 Stunden eine zweite Zugabe von 1,6 g o-Chlor-benzylchlorid und 1,2 g absolutem Triäthylamin erfolgt. Nach dem Erkalten wird die Reaktionslösung mehrmals mit Wasser gewaschen und über Natriumsulfat getrocknet. Nach Einengen der entsprechenden Fraktionen wird der Rückstand in Isopropylalkohol gelöst, mit isopropylalkoholischer Salzsäure das Hydrochlorid gefällt und nach dem Absaugen aus Isopropylalkohol umkristallisiert.

Ausbeute : 6,8 g (48 % der Theorie),
Schmelzpunkt : 195-197 °C
Ber. : C 57,33 H 5,45 N 4,46 Cl 22,56 S 10,20
Gef. : C 57,54 H 5,52 N 4,48 Cl 22,50 S 10,32

Analog den Beispielen 9 und 10 wurden folgende Verbindungen hergestellt :

6-Allyl-5,6,7,8-tetrahydro-4H-thieno [2,3-d] azepin-hydrochlorid

Ausbeute : 23,8 % der Theorie,
Schmelzpunkt : 204-206 °C
Ber. : C 57,50 H 7,02 N 6,10 Cl 15,43 S 13,95
Gef. : C 57,40 H 6,94 N 6,13 Cl 15,55 S 14,05

6-Äthyl-5,6,7,8-tetrahydro-4H-thieno [2,3-d] azepin-hydrochlorid

Ausbeute : 33 % der Theorie,
Schmelzpunkt : 231-234 °C
Ber. : C 55,16 H 7,41 N 6,43 Cl 16,28 S 14,72
Gef. : C 54,90 H 7,50 N 6,45 Cl 16,05 S 14,87

5,6,7,8-Tetrahydro-4H-thieno [2,3-d] azepin-hydrochlorid

Ausbeute : 32 % der Theorie,
Schmelzpunkt : 211-213 °C
Ber. : C 50,65 H 6,38 N 7,38 Cl 18,69 S 16,90
Gef. : C 50,70 H 6,40 N 7,39 Cl 18,60 S 16,80

6-(2-Chlor-benzoyl)-5,6,7,8-tetrahydro-4H-thieno [2,3-d] azepin

Ausbeute : 75 % der Theorie,
Schmelzpunkt : 113-115 °C
Ber. : C 61,74 H 4,84 N 4,80 Cl 12,15 S 10,99
Gef. : C 61,84 H 4,97 N 4,88 Cl 12,17 S 11,00

Beispiel 11

6-(2,6-Dichlorbenzyl)-5,6,7,8-tetrahydro-4H-thieno [2,3-d] azepin-hydrochlorid

Hergestellt analog Beispiel 10 aus 5,6,7,8-Tetrahydro-4H-thieno [2,3-d] azepin und 2,6-Dichlorbenzylbromid.

Ausbeute : 58 % der Theorie,
Schmelzpunkt : 213 °C
Ber. : C 51,66 H 4,62 Cl 30,50 N 4,02 S 9,19
Gef. : C 51,73 H 4,48 Cl 30,60 N 4,00 S 9,14

Beispiel 12

6-(2,4-Dichlorbenzyl)-5,6,7,8-tetrahydro-4H-thieno [2,3-d] azepin-hydrochlorid

Hergestellt analog Beispiel 10 aus 5,6,7,8-Tetrahydro-4H-thieno [2,3-d] azepin und 2,4-Dichlorbenzylchlorid.
Ausbeute : 56 % der Theorie,
Schmelzpunkt : 181-182 °C
Ber. : C 51,66 H 4,62 Cl 30,50 N 4,02 S 9,19
Gef. : C 51,90 H 4,47 Cl 30,10 N 3,93 S 9,25

Beispiel 13

6-Benzyl-5,6,7,8-tetrahydro-4H-1-methyl-pyrrolo [2,3-d] azepin-2-carbonsäure-äthylester-hydrochlorid

8,6 g (0,03 Mol) 1-Benzyl-4-chlor-5-formyl-2,3,6,7-tetrahydro-1H-azepin-hydrochlorid werden mit 9,2 g (0,06 Mol) Sarcosin-äthylester-hydrochlorid und 12,7 g (0,12 Mol) wasserfreiem Soda in 90 ml absolutem Dimethylsulfoxid 1,5 Stunden bei 40 °C gerührt. Danach wird auf ca. 300 ml Eiswasser gegossen und 2 mal mit Essigsäureäthylester extrahiert. Die vereinigten Extrakte werden 3 mal mit Wasser gewaschen, über Natriumsulfat getrocknet und eingedampft. Der so erhaltene rote, ölige Rückstand (5,8 g = 0,017 5 Mol) wird in 20 ml absolutem Dimethylformamid gelöst und bei 5-10 °C unter Stickstoffatmosphäre langsam in eine Lösung von 2,05 g (0,017 5 Mol) Kalium-tert.butylat in 15 ml absolutem Dimethylformamid getropft. Nach halbstündigem Weiterrühren bei Raumtemperatur wird die gelartige Reaktionslösung auf Eiswasser gegeben und mit Essigsäure-äthylester mehrfach extrahiert. Die vereinigten organischen Phasen werden 4 mal mit Wasser gewaschen, über Natriumsulfat getrocknet und eingedampft. Der Rückstand wird über Kieselgel im Fließmittel Toluol/Äthanol = 19 : 1 säulenchromatographisch gereinigt.
Ausbeute : 3,4 g (36 % der Theorie),
Schmelzpunkt : $< 20$ °C
Das Produkt wird in Essigsäureäthylester gelöst, mit ätherischer Salzsäure das Hydrochlorid gefällt, abgesaugt und aus Essigsäureäthylester/Isopropylalkohol (9 : 1) umkristallisiert.
Schmelzpunkt : 202-203 °C (Zers.)
Ber. : C 65,41 H 7,22 Cl 10,16 N 8,03
Gef. : C 65,40 H 7,20 Cl 9,27 N 8,09

Beispiel 14

6-Benzyloxycarbonyl-5,6,7,8-tetrahydro-4H-1-methyl-pyrrolo [2,3-d] azepin-2-carbonsäure-äthylester

Hergestellt analog Beispiel 13 aus 4-Chlor-5-formyl-2,3,6,7-tetrahydro-1H-azepin-carbonsäure-benzylester und Sarcosin-äthylester-hydrochlorid.
Ausbeute : 8 % der Theorie,
Schmelzpunkt : 73-74 °C
Ber. : C 67,00 H 6,79 N 7,86
Gef. : C 67,49 H 6,77 N 7,69

Beispiel 15

6-(2-Chlorbenzyl)-5,6,7,8-tetrahydro-4H-1-methyl-pyrrolo [2,3-d] azepin-2-carbonsäure-äthylester-hydrochlorid

Hergestellt analog Beispiel 3 aus 5,6,7,8-Tetrahydro-4H-1-methyl-pyrrolo [2,3-d] azepin-2-carbonsäure-äthylester-hydrochlorid und 2-Chlorbenzylchlorid.
Ausbeute : 81 % der Theorie,
Schmelzpunkt : 169-171 °C
Ber. : C 59,53 H 6,31 Cl 18,50 N 7,31
Gef. : C 59,47 H 6,34 Cl 18,60 N 7,43

Beispiel 16

6-Äthyl-5,6,7,8-tetrahydro-4H-1-methyl-pyrrolo [2,3-d] azepin-2-carbonsäure-äthylester-hydrochlorid

Hergestellt analog Beispiel 3 aus 5,6,7,8-Tetrahydro-4H-1-methyl-pyrrolo [2,3-d] azepin-2-carbonsäure-äthylester-hydrochlorid und Äthyljodid.
Ausbeute : 62 % der Theorie,
Schmelzpunkt : 188-189 °C

Ber.: C 58,63 H 8,08 Cl 12,36 N 9,77
Gef.: C 58,40 H 8,12 Cl 12,35 N 9,78

Beispiel 17

6-(4-Chlorbenzyl)-5,6,7,8-tetrahydro-4H-1-methyl-pyrrolo [2,3-d] azepin-2-carbonsäure-äthylester-hydrochlorid

Hergestellt analog Beispiel 3 aus 5,6,7,8-Tetrahydro-4H-1-methyl-pyrrolo [2,3-d] azepin-2-carbonsäure-äthylester-hydrochlorid und 4-Chlorbenzylchlorid.
Ausbeute: 55 % der Theorie,
Schmelzpunkt: 185-186 °C
Ber.: C 59,53 H 6,31 Cl 18,50 N 7,31
Gef.: C 59,79 H 6,65 Cl 18,10 N 7,14

Beispiel 18

5,6,7,8-Tetrahydro-4H-1-methyl-pyrrolo [2,3-d] azepin-2-carbonsäure-äthylester-hydrochlorid

Hergestellt analog Beispiel 2 aus 6-Benzyl-5,6,7,8-tetrahydro-4H-1-methyl-pyrrolo [2,3-d] azepin-2-carbonsäure-äthylester und katalytisch erregtem Wasserstoff.
Ausbeute: 84 % der Theorie,
Schmelzpunkt: 179-180 °C
Ber.: C 55,70 H 7,40 Cl 13,70 N 10,83
Gef.: C 55,84 H 7,28 Cl 13,45 N 10,93

Beispiel 19

6-Benzyl-5,6,7,8-tetrahydro-4H-1-methyl-pyrrolo [2,3-d] azepin-2-carbonsäure

3,1 g (0,01 Mol) 6-Benzyl-5,6,7,8-tetrahydro-4H-1-methyl-pyrrolo [2,3-d] azepin-2-carbonsäure-äthylester werden unter Zusatz von 10 ml 2 n Natronlauge in 80 ml Äthanol gelöst und 6 Stunden auf Rückflußtemperatur erhitzt. Es wird eingedampft und der Rückstand über Kieselgel im Fließmittel Chloroform/Methanol (8,5/1,5) säulenchromatographisch gereinigt.
Ausbeute: 0,8 g (29 % der Theorie),
Schmelzpunkt: 149-150 °C (Zers.)
Ber.: C 71,81 H 7,09 N 9,85
Gef.: C 71,94 H 6,88 N 10,09

Beispiel 20

6-(2-Chlorbenzyl)-5,6,7,8-tetrahydro-4H-1-methyl-pyrrolo [2,3-d] azepin-hydrochlorid

Das analog Beispiel 19 durch Verseifung von 3,3 g (0,009 5 Mol) 6-(2-Chlorbenzyl)-5,6,7,8-tetrahydro-4H-1-methyl-pyrrolo [2,3-d] azepin-2-carbonsäure-äthylester erhaltene Natriumsalz wird mit 3,1 g (0,035 Mol) Oxalsäure in 50 ml Propanol 8 Stunden auf Rückflußtemperatur erhitzt. Danach wird eingedampft, der Rückstand mit Wasser verrührt und mit 2 n Natronlauge alkalisch gestellt. Nach 3-maligem Extrahieren mit Chloroform werden die vereinigten organischen Phasen mit Wasser gewaschen, über Natriumsulfat getrocknet und eingedampft. Der Rückstand wird über Kieselgel im Fließmittel Toluol/Essigsäureäthylester äthanolisches Ammoniak (9/1/0,05) säulenchromatographisch gereinigt. Das nach Eindampfen des Lösungsmittels zurückbleibende gelbliche Öl wird in absolutem Äther gelöst und mit ätherischer Salzsäure das Hydrochlorid gefällt, das nach Absaugen nochmals durch Umfällen mit Äther und heißem Isopropanol gereinigt wird.
Ausbeute: 1,9 g (64 % der Theorie),
Schmelzpunkt: 179-180 °C
Ber.: C 61,74 H 6,48 Cl 22,78 N 9,00
Gef.: C 61,67 H 6,46 Cl 22,55 N 8,99

Beispiel 21

6-Benzyl-5,6,7,8-tetrahydro-4H-1-methyl-pyrrolo [2,3-d] azepin-oxalat

Hergestellt analog Beispiel 20 aus 6-Benzyl-5,6,7,8-tetrahydro-4H-1-methyl-pyrrolo [2,3-d] azepin-2-carbonsäure-äthylester durch Verseifung und Decarboxylierung.
Ausbeute: 64 % der Theorie,

Schmelzpunkt : 182-183 °C
Ber. : C 65,44 H 6,71 N 8,48
Gef. : C 65,54 H 6,76 N 8,44

Beispiel 22

6-(4-Chlorbenzyl)-5,6,7,8-tetrahydro-4H-1-methyl-pyrrolo [2,3-d] azepin-oxalat

Hergestellt analog Beispiel 20 aus 6-(4-Chlorbenzyl)-5,6,7,8-tetrahydro-4H-1-methyl-pyrrolo [2,3-d] azepin-2-carbonsäure-äthylester durch Verseifung und Decarboxylierung.
Ausbeute : 29 % der Theorie,
Schmelzpunkt : 195-196 °C
Ber. : C 59,26 H 5,80 Cl 9,72 N 7,68
Gef. : C 59,20 H 5,86 Cl 9,56 N 7,51

Beispiel 23

5,6,7,8-Tetrahydro-4H-1-methyl-pyrrolo [2,3-d] azepin-oxalat

Hergestellt analog Beispiel 20 aus 5,6,7,8-Tetrahydro-4H-1-methyl-pyrrolo [2,3-d] azepin-2-carbonsäure-äthylester durch Verseifung und Decarboxylierung.
Ausbeute : 29 % der Theorie,
Schmelzpunkt : 180-181 °C
Ber. : C 54,99 H 6,71 N 11,66
Gef. : C 55,18 H 6,75 N 11,36

Beispiel 24

6-(2-Chlorbenzyl)-5,6,7,8-tetrahydro-4H-furo [2,3-d] azepin-2-carbonsäure-butylester

In eine Suspension aus 0,72 g (0,015 Mol) 50 %igem Natriumhydrid und 10 ml absolutem Dioxan werden bei Raumtemperatur unter Einleiten von trockenem Stickstoff 2,0 g (0,015 Mol) Glykolsäurebutylester unter starkem Rühren langsam eingetropft. Die Temperatur der stark aufschäumenden Reaktionsmischung wird dabei durch gelegentliches Abkühlen mit Eiswasser unterhalb 30 °C gehalten. Nach 1,5 Stunden weiteren Rührens bei Raumtemperatur wird eine Lösung von 2,84 g (0,010 Mol) 1-(2-Chlorbenzyl)-4-chlor-5-formyl-2,3,6,7-tetrahydro-1H-azepin in 20 ml absolutem Dioxan zugetropft, wobei die Reaktionstemperatur von 20 °C auf ca. 33 °C ansteigt. Danach wird nochmals 2 Stunden bei Raumtemperatur weitergerührt, auf ca. 150 ml Eiswasser gegossen und 3 mal mit Essigsäureäthylester extrahiert. Die vereinigten organischen Phasen werden 3 mal mit Wasser geschüttelt, über Natriumsulfat getrocknet und eingedampft. Der Rückstand wird über Kieselgel im Fließmittel Chloroform/Essigsäureäthylester = 93 : 7 säulenchromatographisch gereinigt.
Ausbeute : 0,2 g (6 % der Theorie),
Schmelzpunkt : 58 °C
Ber. : C 66,38 H 6,68 Cl 9,80 N 3,87
Gef. : C 66,31 H 6,50 Cl 9,93 N 3,78

Beispiel 25

6-(2-Chlorbenzyl)-5,6,7,8-tetrahydro-4H-furo [2,3-d] azepin-2-carbonsäure

Hergestellt analog Beispiel 19 durch Verseifung von 6-(2-Chlor-benzyl)-5,6,7,8-tetrahydro-4H-furo [2,3-d] azepin-2-carbonsäure-butylester mit wässriger Natronlauge in Äthanol.
Ausbeute : 65 % der Theorie,
Schmelzpunkt : 120 °C (Zers., sintern ab 200 °C)
Ber. : Molpeak m/e = 305/307 (1 Cl)
Gef. : Molpeak m/e = 305/307 (1 Cl)
Ber. : C 62,85 H 5,27 Cl 11,60 N 4,58
Gef. : C 62,66 H 5,35 Cl 11,39 N 4,71

Beispiel 26

6-(2-Chlorbenzyl)-5,6,7,8-tetrahydro-4H-furo [2,3-d] azepin

Hergestellt analog Beispiel 20 aus 6-(2-Chlorbenzyl)-5,6,7,8-tetrahydro-4H-furo [2,3-d] azepin-2-carbonsäure durch Decarboxylierung.

Ausbeute : 35 % der Theorie,
Schmelzpunkt : < 20 °C
Ber. : Molpeak m/e = 261/263 (1 Cl)
Gef. : Molpeak m/e = 261/263 (1 Cl)
Ber. : C 68,83 H 6,16 Cl 13,54 N 5,35
Gef. : C 68,79 H 6,33 Cl 13,36 N 5,62

### Beispiel 27

Suppositorien zu 30 mg 6-Benzyl-5,6,7,8-tetrahydro-4H-thieno [2,3-d] azepin-2-carbonsäure-hydrochlorid

Zusammensetzung :
1 Zäpfchen enthält :

| | |
|---|---|
| Wirksubstanz | 0,030 g |
| Zäpfchenmasse (z. B. Witepsol W 45 | 1,336 g |
| und Witepsol E 75) | 0,334 g |
| | 1,700 g |

### Herstellung

Der gemahlene Wirkstoff wird unter Rühren in das auf 40 °C temperierte aufgeschmolzene Gemisch der Zäpfchenmassen eingetragen und die Schmelze in gekühlte Gießformen ausgegossen. Nach dem völligen Erstarren werden die Suppositorien den Formen entnommen und in geeigneter Weise verpackt.

### Beispiel 28

Gelatine-Steckkapseln zu 5 mg 6-Benzyl-5,6,7,8-tetrahydro-4H-thieno [2,3-d] azepin-2-carbonsäure-hydrochlorid

1 Kapsel enthält :

| | |
|---|---|
| Wirksubstanz | 5,0 mg |
| Maisstärke getr. | 100,0 mg |
| Maisstärke pulv. | 93,0 mg |
| Magnesiumstearat | 2,0 mg |
| | 200,0 mg |

### Herstellung

Wirkstoff und Hilfsstoffe werden gemischt, durch ein Sieb der Maschenweite 0,75 mm gegeben und in einem geeigneten Mischer homogen verteilt. Das Pulver wird auf einem Kapselfüll- und Schließautomaten in Hartgelatine-Steckkapseln der Größe 3 (Parke Davis) abgefüllt.

### Beispiel 29

Tabletten zu 25 mg 6-Benzyl-5,6,7,8-tetrahydro-4H-thieno [2,3-d] azepin-2-carbonsäure-hydrochlorid

1 Tablette enthält :

| | |
|---|---|
| Wirksubstanz | 25,0 mg |
| Milchzucker | 35,0 mg |
| Maisstärke | 15,0 mg |
| Polyvinylpyrrolidon | 4,5 mg |
| Magnesiumstearat | 0,5 mg |
| | 80,0 mg |

### Herstellung

Der Wirkstoff wird mit Milchzucker und Stärke gemischt und danach mit der wäßrigen Polyvinylpyrrolidon-Lösung gleichmäßig befeuchtet.
Feuchtsiebung : 1,5 mm-Maschenweite
Trocknung : Umlufttrockenschrank bei 45 °C
Trockensiebung : 1,0 mm-Maschenweite
Das trockene Granulat wird nach Zumischung des Schmiermittels zu Tabletten verpreßt.
Tabletten : 6 mm Durchmesser, beidseitige Facette, einseitige Teilkerbe, biplan.

Beispiel 30

Dragées zu 25 mg 6-Benzyl-5,6,7,8-tetrahydro-4H-thieno [2,3-d] azepin-2-carbonsäure-hydrochlorid

Die Herstellung der preßfertigen Mischung erfolgt analog Beispiel 28.

Verpressung zu bikonvexen Dragéekernen von 80,0 mg Gewicht, 6 mm Durchmesser und Wölbungsradius 5 mm.

Die Kerne werden mit einer üblichen Zuckerdragiersuspension auf ein Gewicht von 110 mg im Dragierkessel dragiert und anschließend mit einer Poliersuspension poliert.

Beispiel 31

Tabletten zu 300 mg 6-(2-Chlorbenzyl)-5,6,7,8-tetrahydro-4H-thieno [2,3-d] azepin-hydrochlorid

Zusammensetzung :

1 Tablette enthält :

| | |
|---|---|
| Wirksubstanz | 300,0 mg |
| Milchzucker | 120,0 mg |
| Cellulose mikrokristallin | 100,0 mg |
| Maisstärke | 72,0 mg |
| Polyvinylpyrrolidon | 6,0 mg |
| Magnesiumstearat | 2,0 mg |
| | 600,0 mg |

Herstellung

Die Wirksubstanz wird mit Milchzucker, Cellulose und Maisstarke gemischt und mit einer 15 %igen Lösung von Polyvinylpyrrolidon in Wasser granuliert. Die feuchte Masse wird durch ein Sieb geschlagen, auf Horden ausgebreitet und bei 45 °C getrocknet. Nach einer nochmaligen Siebung wird Magnesiumstearat zugemischt und die Mischung zu Tabletten verpreßt.

Tablettengewicht : 600 mg

Stempel : 13 mm Durchmesser, biplan beidseitige Facette und einseitige Teilkerbe

Beispiel 32

Dragées zu 250 mg 6-(2-Chlorbenzyl)-5,6,7,8-tetrahydro-4H-thieno [2,3-d] azepin-hydrochlorid

1 Dragéekern enthält :

| | |
|---|---|
| Wirksubstanz | 250,0 mg |
| Milchzucker | 100,0 mg |
| Cellulose mikrokristallin | 40,0 mg |
| Maisstärke | 84,0 mg |
| Polyvinylpyrrolidon | 5,0 mg |
| Magnesiumstearat | 1,0 mg |
| | 480,0 mg |

Herstellung

Die Herstellung der preßfertigen Mischung erfolgt analog Beispiel 31.

Kerngewicht : 480 mg

Stempel : 11 mm gewölbt mit einem Wölbungsradius von 10 mm.

Die Dragéekerne werden auf bekannte Art in einem Dragéekessel mit einer Zuckerschicht überzogen.

Dragéegewicht : 560 mg

Beispiel 33

Suppositorien zu 400 mg 6-(2-Chlorbenzyl)-5,6,7,8-tetrahydro-4H-thieno [2,3-d] azepin-hydrochlorid

1 Zäpfchen enthält :

| | |
|---|---|
| Wirksubstanz | 0,40 g |
| Zäpfchenmasse (z. B. Witepsol H 19 und Witepsol W 45) | 1,30 g |
| | 1,70 g |

Herstellung

Das Hartfett wird geschmolzen. Bei 38 °C wird die gemahlene Wirksubstanz in der Schmelze homogen dispergiert. Es wird auf 35 °C abgekühlt und in schwach vorgekühlte Suppositorienformen ausgegossen.

Zäpfchengewicht : 1,7 g

**Patentansprüche** (für die Vertragsstaaten : BE, CH, DE, FR, IT, LI, LU, NL, SE)

1. Azepinderivate der allgemeinen Formel (I)

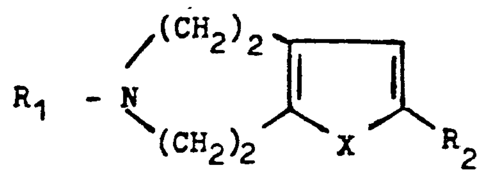

(I)

in der

$R_1$ ein Wasserstoffatom, eine Alkylgruppe mit 1 bis 12 Kohlenstoffatomen, eine Allylgruppe, eine gegebenenfalls im aromatischen Kern durch Hydroxygruppen und/oder Halogenatome mono- oder disubstituierte Aralkylgruppe mit 7 bis 9 Kohlenstoffatomen, eine gegebenenfalls durch Halogenatome und/oder Alkoxygruppen mit 1 bis 3 Kohlenstoffatomen mono- oder disubstituierte Benzoylgruppe, eine Alkoxycarbonylgruppe mit insgesamt 2 bis 4 Kohlenstoffatomen oder eine Aralkoxycarbonylgruppe mit insgesamt 8 bis 10 Kohlenstoffatomen,

$R_2$ ein Wasserstoffatom, eine Carboxygruppe oder eine Alkoxycarbonylgruppe mit insgesamt 2 bis 4 Kohlenstoffatomen und

X ein Sauerstoff- oder Schwefelatom oder eine Iminogruppe der Formel

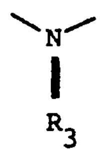

bedeuten, wobei $R_3$ ein Wasserstoffatom, eine gegebenenfalls durch eine Phenylgruppe substituierte Alkylgruppe mit 1 bis 3 Kohlenstoffatomen oder eine Phenylgruppe darstellt, und deren Salze mit anorganischen oder organischen Säuren oder Basen, wenn $R_2$ eine Carboxygruppe darstellt, insbesondere deren physiologisch verträgliche Salze.

2. Verbindungen der allgemeinen Formel (I) gemäß Anspruch 1, in der

X ein Sauerstoff- oder Schwefelatom, eine Imino-, Methylimino-, Phenylimino- oder Benzyliminogruppe,

$R_1$ ein Wasserstoffatom, eine Alkylgruppe mit 1 bis 3 Kohlenstoffatomen, eine gegebenenfalls im Phenylkern durch Chlor- oder Bromatome mono- oder disubstituierte Benzylgruppe, eine im Phenylkern durch eine Hydroxygruppe und ein Chlor- oder Bromatom substituierte Benzylgruppe, eine gegebenenfalls durch ein Chlor- oder Bromatom substituierte Benzoylgruppe, eine Alkoxycarbonylgruppe mit insgesamt 2 bis 4 Kohlenstoffatomen, eine Allyl-, Dodecyl-, Chlor-methoxybenzoyl- oder Benzyloxycarbonylgruppe,

$R_2$ ein Wasserstoffatom, eine Carboxygruppe oder eine Alkoxycarbonylgruppe mit insgesamt 2 bis 4 Kohlenstoffatomen bedeuten, und deren Salze mit anorganischen oder organischen Säuren oder Basen, wenn $R_2$ eine Carboxygruppe darstellt, insbesondere deren physiologisch verträgliche Salze.

3. Verbindungen der allgemeinen Formel (I) gemäß Anspruch 1,
in der

X ein Schwefelatom,

$R_1$ eine Dodecyl-, Benzyl-, Chlorbenzyl- oder Chlorbenzoylgruppe und

$R_2$ ein Wasserstoffatom oder die Carboxygruppe bedeuten, und deren physiologisch verträgliche Salze mit anorganischen oder organischen Säuren oder Basen.

4. 6-(2-Chlorbenzyl)-5,6,7,8-tetrahydro-4H-thieno [2,3-d] azepin und dessen physiologisch verträgliche Säureadditionssalze.

5. 6-Benzyl-5,6,7,8-tetrahydro-4H-thieno [2,3-d] azepin-2-carbonsäure und dessen physiologisch verträgliche Salze.

6. Arzneimittel, enthaltend eine Verbindung gemäß den Ansprüchen 3 bis 5 oder dessen physiologisch verträgliches Salz neben einem oder mehreren inerten Trägerstoffen und/oder Verdünnungsmitteln.

7. Verbindungen gemäß den Ansprüchen 3 bis 5 oder dessen physiologisch verträgliche Salze zur Verwendung bei der Bekämpfung von thromboembolischen Erkrankungen und Erkrankungen des Intermediärstoffwechsels.

8. Verfahren zur Herstellung von Verbindungen gemäß den Ansprüchen 1 bis 5, dadurch gekennzeichnet, daß eine gegebenenfalls im Reaktionsgemisch gebildete Verbindung der allgemeinen Formel (II)

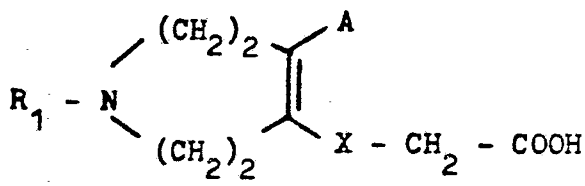

(II)

in der

$R_1$ und X wie in den Ansprüchen 1 bis 5 definiert sind und

A die Formylgruppe oder deren Acetale darstellt, oder deren Ester cyclisiert und gegebenenfalls die erhaltene Verbindung anschließend hydrolysiert und/oder decarboxyliert wird und gewünschtenfalls anschließend eine erhaltene Verbindung der allgemeinen Formel (I), in der $R_1$ eine gegebenenfalls im aromatischen Kern durch Hydroxygruppen und/oder Halogenatome mono- oder disubstituierte Benzylgruppe und/oder X eine Benzyliminogruppe darstellt, mittels katalytischer Hydrierung in eine entsprechende Verbindung der allgemeinen Formel (I), in der $R_1$ ein Wasserstoffatom und/oder X eine Iminogruppe darstellt, übergeführt wird oder eine erhaltene Verbindung der allgemeinen Formel (I), in der $R_1$ eine gegebenenfalls im aromatischen Kern durch Hydroxygruppen und/oder Halogenatome mono- oder disubstituierte Benzylgruppe darstellt, mittels Raney-Nickel in Gegenwart eines entsprechenden Alkohols in eine entsprechende Verbindung der allgemeinen Formel (I), in der $R_1$ eine Alkylgruppe mit 1 bis 12 Kohlenstoffatomen darstellt, übergeführt wird oder eine erhaltene Verbindung der allgemeinen Formel I, in der $R_1$ und/oder $R_2$ eine Alkoxycarbonylgruppe mit insgesamt 2 bis 4 Kohlenstoffatomen darstellt, mittels Hydrolyse unter gleichzeitiger Decarboxylierung, falls $R_1$ eine Alkoxycarbonylgruppe mit insgesamt 2 bis 4 Kohlenstoffatomen darstellt, in eine entsprechende Verbindung der allgemeinen Formel (I), in der $R_1$ ein Wasserstoffatom und/oder $R_2$ eine Carboxygruppe darstellt, übergeführt wird oder eine erhaltene Verbindung der allgemeinen Formel (I) in ihre Salze, insbesondere in ihre physiologisch verträglichen Salze mit einer anorganischen oder organischen Säure oder Base, übergeführt wird.

9. Verfahren zur Herstellung von Verbindungen gemäß den Ansprüchen 1 bis 5, in denen $R_2$ ein Wasserstoffatom darstellt und $R_1$ mit Ausnahme der Alkoxycarbonylgruppe die in den Ansprüchen 1 bis 5 angegebenen Bedeutungen hat, dadurch gekennzeichnet, daß eine gegebenenfalls im Reaktionsgemisch gebildete Carbonsäure der allgemeinen Formel (III)

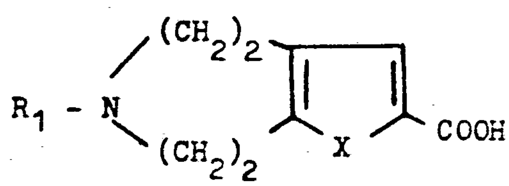

(III)

in der $R_1$ und X wie in den Ansprüchen 1 bis 5 definiert sind, decarboxyliert wird und gewünschtenfalls anschließend eine erhaltene Verbindung der allgemeinen Formel I, in der $R_1$ eine gegebenenfalls im aromatischen Kern durch Hydroxygruppen und/oder Halogenatome mono- oder disubstituierte Benzylgruppe und/oder X eine Benzyliminogruppe darstellt, mittels katalytischer Hydrierung in eine entsprechende Verbindung der allgemeinen Formel (I), in der $R_1$ ein Wasserstoffatom und/oder X eine Iminogruppe darstellt, übergeführt wird oder eine erhaltene Verbindung der allgemeinen Formel (I), in der $R_1$ eine gegebenenfalls im aromatischen Kern durch Hydroxygruppen und/oder Halogenatome mono- oder disubstituierte Benzylgruppe darstellt, mittels Raney-Nickel in Gegenwart eines entsprechenden Alkohols in eine entsprechende Verbindung der allgemeinen Formel (I), in der $R_1$ eine Alkylgruppe mit 1 bis 12 Kohlenstoffatomen darstellt, übergeführt wird oder eine erhaltene Verbindung der allgemeinen Formel (I), in der $R_1$ und/oder $R_2$ eine Alkoxycarbonylgruppe mit insgesamt 2 bis 4 Kohlenstoffatomen darstellt, mittels Hydrolyse unter gleichzeitiger Decarboxylierung, falls $R_1$ eine Alkoxycarbonylgruppe mit insgesamt 2 bis 4 Kohlenstoffatomen darstellt, in eine entsprechende Verbindung der allgemeinen Formel (I), in der $R_1$ ein Wasserstoffatom und/oder $R_2$ eine Carboxygruppe darstellt, übergeführt wird oder

eine erhaltene Verbindung der allgemeinen Formel (I) in ihre Salze, insbesondere in ihre physiologisch verträglichen Salze mit einer anorganischen oder organischen Säure, übergeführt wird.

10. Verfahren zur Herstellung von Verbindungen gemäß den Ansprüchen 1 bis 5, in denen $R_1$ mit Ausnahme des Wasserstoffatoms die in den Ansprüchen 1 bis 5 angegebenen Bedeutungen hat, dadurch gekennzeichnet, daß eine Verbindung der allgemeinen Formel (IV)

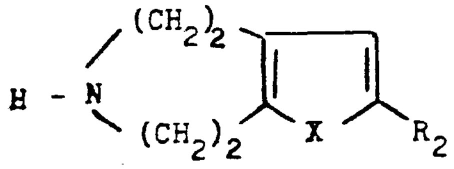

(IV)

in der $R_2$ und X wie in den Ansprüchen 1 bis 5 definiert sind, mit einer Verbindung der allgemeinen Formel (V)

$$R_1—Z \qquad (V)$$

in der

$R_1$ mit Ausnahme des Wasserstoffatoms wie in den Ansprüchen 1 bis 5 definiert ist und

Z eine nukleophile Austrittsgruppe, eine Hydroxygruppe oder zusammen mit einem Wasserstoffatom des benachbarten Kohlenstoffatoms ein Sauerstoffatom darstellt, umgesetzt wird

und gewünschtenfalls anschließend eine erhaltene Verbindung der allgemeinen Formel (I), in der X eine Benzyliminogruppe darstellt, mittels katalytischer Hydrierung in eine entsprechende Verbindung der allgemeinen Formel (I), in der X eine Iminogrupppe darstellt, übergeführt wird oder eine erhaltene Verbindung der allgemeinen Formel (I), in der $R_1$ eine gegebenenfalls im aromatischen Kern durch Hydroxygruppen und/oder Halogenatome mono- oder disubstituierte Benzylgruppe darstellt, mittels Raney-Nickel in Gegenwart eines entsprechenden Alkohols in eine entsprechende Verbindung der allgemeinen Formel (I), in der $R_1$ eine Alkylgruppe mit 1 bis 12 Kohlenstoffatomen darstellt, übergeführt wird oder

eine erhaltene Verbindung der allgemeinen Formel (I), in der $R_2$ eine Alkoxycarbonylgruppe mit insgesamt 2 bis 4 Kohlenstoffatomen darstellt, mittels Hydrolyse, in eine entsprechende Verbindung der allgemeinen Formel (I), in der $R_2$ eine Carboxygruppe darstellt, übergeführt wird oder

eine erhaltene Verbindung der allgemeinen Formel (I) in ihre Salze, insbesondere in ihre physiologisch verträglichen Salze mit einer anorganischen oder organischen Säure oder Base, übergeführt wird.

**Patentansprüche** (für den Vertragsstaat AT)

1. Verfahren zur Herstellung von Azepinderivaten der allgemeinen Formel I

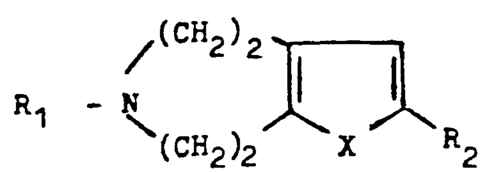

(I)

in der

$R_1$ ein Wasserstoffatom, eine Alkylgruppe mit 1 bis 12 Kohlenstoffatomen, eine Allylgruppe, eine gegebenenfalls im aromatischen Kern durch Hydroxygruppen und/oder Halogenatome mono- oder disubstituierte Aralkylgruppe mit 7 bis 9 Kohlenstoffatomen, eine gegebenenfalls durch Halogenatome und/oder Alkoxygruppen mit 1 bis 3 Kohlenstoffatomen mono- oder disubstituierte Benzoylgruppe, eine Alkoxycarbonylgruppe mit insgesamt 2 bis 4 Kohlenstoffatomen oder eine Aralkoxycarbonylgruppe mit insgesamt 8 bis 10 Kohlenstoffatomen,

$R_2$ ein Wasserstoffatom, eine Carboxygruppe oder eine Alkoxycarbonylgruppe mit insgesamt 2 bis 4 Kohlenstoffatomen und

X ein Sauerstoff- oder Schwefelatom oder eine Iminogruppe der Formel

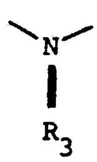

bedeuten, wobei $R_3$ ein Wasserstoffatom, eine gegebenenfals durch eine Phenylgruppe substituierte Alkylgruppe mit 1 bis 3 Kohlenstoffatomen oder eine Phenylgruppe darstellt, sowie von deren Salzen mit anorganischen oder organischen Säuren oder Basen, wenn $R_2$ eine Carboxygruppe darstellt, insbesondere von deren physiologisch verträglichen Salzen, dadurch gekennzeichnet, daß eine gegebenenfalls im Reaktionsgemisch gebildete Verbindung der allgemeinen Formel (II)

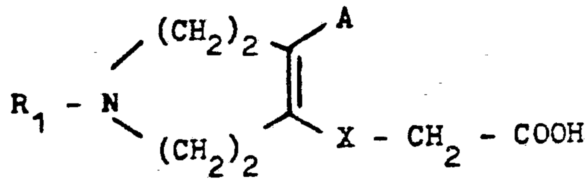

(II)

in der
$R_1$ und X eingangs definiert ist und
A die Formylgruppe oder deren Acetale darstellt, oder deren Ester cyclisiert und gegebenenfalls die erhaltene Verbindung anschließend hydrolysiert und/oder decarboxyliert wird
und gewünschtenfalls anschließend eine erhaltene Verbindung der allgemeinen Formel (I), in der $R_1$ eine gegebenenfalls im aromatischen Kern durch Hydroxygruppen und/oder Halogenatome mono- oder disubstituierte Benzylgruppe und/oder X eine Benzyliminogruppe darstellt, mittels katalytischer Hydrierung in eine entsprechende Verbindung der allgemeinen Formel (I), in der $R_1$ ein Wasserstoffatom und/oder X eine Iminogruppe darstellt, übergeführt wird oder eine erhaltene Verbindung der allgemeinen Formel (I), in der $R_1$ eine gegebenenfalls im aromatischen Kern durch Hydroxygruppen und/oder Halogenatome mono- oder disubstituierte Benzylgruppe darstellt, mittels Raney-Nickel in Gegenwart eines entsprechenden Alkohols in eine entsprechende Verbindung der allgemeinen Formel (I), in der $R_1$ eine Alkylgruppe mit 1 bis 12 Kohlenstoffatomen darstellt, übergeführt wird oder
eine erhaltene Verbindung der allgemeinen Formel (I), in der $R_1$ und/oder $R_2$ eine Alkoxycarbonylgruppe mit insgesamt 2 bis 4 Kohlenstoffatomen darstellt, mittels Hydrolyse unter gleichzeitiger Decarboxylierung, falls $R_1$ eine Alkoxycarbonylgruppe mit insgesamt 2 bis 4 Kohlenstoffatomen darstellt, in eine entsprechende Verbindung der allgemeinen Formel (I), in der $R_1$ ein Wasserstoffatom und/oder $R_2$ eine Carboxygruppe darstellt, übergeführt wird oder
eine erhaltene Verbindung der allgemeinen Formel (I) in ihre Salze, insbesondere in ihre physiologisch verträglichen Salze mit einer anorganischen oder organischen Säure oder Base, übergeführt wird.

2. Verfahren zur Herstellung von Verbindungen der allgemeinen Formel (I) gemäß Anspruch 1, in der $R_2$ ein Wasserstoffatom darstellt und $R_1$ mit Ausnahme der Alkoxycarbonylgruppe die im Anspruch 1 angegebenen Bedeutungen hat, dadurch gekennzeichnet, daß eine gegebenenfalls im Reaktionsgemisch gebildete Carbonsäure der allgemeinen Formel (III)

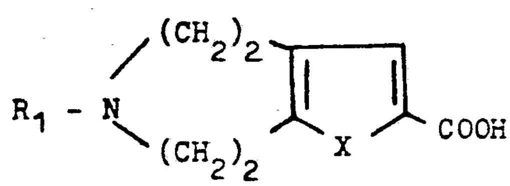

(III)

in der $R_1$ und X wie im Anspruch 1 definiert ist, decarboxyliert wird
und gewünschtenfalls anschließend eine erhaltene Verbindung der allgemeinen Formel (I), in der $R_1$ eine gegebenenfalls im aromatischen Kern durch Hydroxygruppen und/oder Halogenatome mono- oder disubstituierte Benzylgruppe und/oder X eine Benzyliminogruppe darstellt, mittels katalytischer Hydrierung in eine entsprechende Verbindung der allgemeinen Formel (I), in der $R_1$ ein Wasserstoffatom und/oder X eine Iminogruppe darstellt, übergeführt wird oder eine erhaltene Verbindung der allgemeinen Formel (I), in der $R_1$ eine gegebenenfalls im aromatischen Kern durch Hydroxygruppen und/oder Halogenatome mono- oder disubstituierte Benzylgruppe darstellt, mittels Raney-Nickel in Gegenwart eines entsprechenden Alkohols in eine entsprechende Verbindung der allgemeinen Formel I, in der $R_1$ eine Alkylgruppe mit 1 bis 12 Kohlenstoffatomen darstellt, übergeführt wird oder
eine erhaltene Verbindung der allgemeinen Formel (I), in der $R_1$ und/oder $R_2$ eine Alkoxycarbonylgruppe mit insgesamt 2 bis 4 Kohlenstoffatomen darstellt, mittels Hydrolyse unter gleichzeitiger Decarboxylierung, falls $R_1$ eine Alkoxycarbonylgruppe mit insgesamt 2 bis 4 Kohlenstoffatomen darstellt, in eine entsprechende Verbindung der allgemeinen Formel (I), in der $R_1$ ein Wasserstoffatom und/oder $R_2$ eine Carboxygruppe darstellt, übergeführt wird oder
eine erhaltene Verbindung der allgemeinen Formel (I) in ihre Salze, insbesondere in ihre physiologisch verträglichen Salze mit einer anorganischen oder organischen Säure, übergeführt wird.

3. Verfahren zur Herstellung von Verbindungen der allgemeinen Formel (I) gemäß Anspruch 1, in der

$R_1$ mit Ausnahme des Wasserstoffatoms die im Anspruch 1 angegebenen Bedeutungen hat, dadurch gekennzeichnet, daß eine Verbindung der allgemeinen Formel (IV)

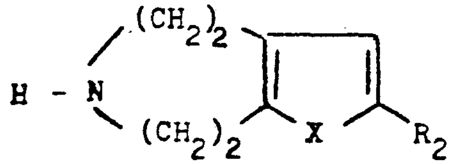

(IV)

in der $R_2$ und X wie im Anspruch 1 definiert sind, mit einer Verbindung der allgemeinen Formel (V)

$R_1'$—Z (V)

in der

$R_1$ mit Ausnahme des Wasserstoffatoms wie im Anspruch 1 definiert ist und

Z eine nukleophile Austrittsgruppe, eine Hydroxygruppe oder zusammen mit einem Wasserstoffatom des benachbarten Kohlenstoffatoms ein Sauerstoffatom darstellt, umgesetzt wird

und gewünschtenfalls anschließend eine erhaltene Verbindung der allgemeinen Formel (I), in der X eine Benzyliminogruppe darstellt, mittels katalytischer Hydrierung in eine entsprechende Verbindung der allgemeinen Formel (I), in der X eine Iminogruppe darstellt, übergeführt wird oder eine erhaltene Verbindung der allgemeinen Formel (I), in der $R_1$ eine gegebenenfalls im aromatischen Kern durch Hydroxygruppen und/oder Halogenatome mono- oder disubstituierte Benzylgruppe darstellt, mittels Raney-Nickel in Gegenwart eines entsprechenden Alkohols in eine entsprechende Verbindung der allgemeinen Formel (I), in der $R_1$ eine Alkylgruppe mit 1 bis 12 Kohlenstoffatomen darstellt, übergeführt wird oder

eine erhaltene Verbindung der allgemeinen Formel I, in der $R_2$ eine Alkoxycarbonylgruppe mit insgesamt 2 bis 4 Kohlenstoffatomen darstellt, mittels Hydrolyse in eine entsprechende Verbindung der allgemeinen Formel (I), in der $R_2$ eine Carboxygruppe darstellt, übergeführt wird oder

eine erhaltene Verbindung der allgemeinen Formel (I) in ihre Salze, insbesondere in ihre physiologisch verträglichen Salze mit einer anorganischen oder organischen Säure oder Base, übergeführt wird.

4. Verfahren zur Herstellung von Verbindungen der allgemeinen Formel (Ia)

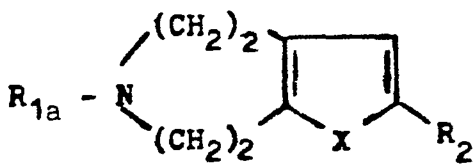

(Ia)

in der

$R_2$ und X wie im Anspruch 1 definiert sind und

$R_{1a}$ ein Wasserstoffatom, eine Alkylgruppe mit 1 bis 12 Kohlenstoffatomen, eine Allylgruppe, eine gegebenenfalls im aromatischen Kern durch ein Halogenatom substituierte Aralkylgruppe mit 7 bis 9 Kohlenstoffatomen, eine gegebenenfalls durch ein Halogenatom und/oder eine Alkoxygruppe mit 1 bis 3 Kohlenstoffatomen mono- oder disubstituierte Benzoylgruppe, eine Alkoxycarbonylgruppe mit insgesamt 2 bis 4 Kohlenstoffatomen oder eine Aralkoxycarbonylgruppe mit insgesamt 8 bis 10 Kohlenstoffatomen bedeutet, dadurch gekennzeichnet, daß eine gegebenenfalls im Reaktionsgemisch gebildete Verbindung der allgemeinen Formel (IIa)

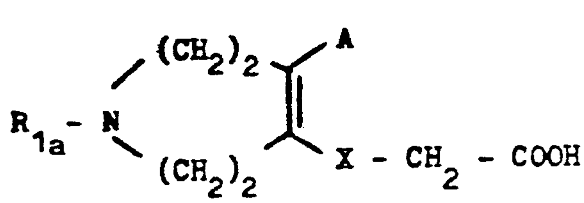

(IIa)

in der $R_{1a}$ wie eingangs definiert ist, oder deren Ester cyclisiert und gegebenenfalls die erhaltene Verbindung anschließend hydrolysiert und/oder decarboxyliert

und gewünschtenfalls anschließend eine erhaltene Verbindung der allgemeinen Formel (Ia), in der $R_{1a}$ eine gegebenenfalls im aromatischen Kern durch ein Halogenatom substituierte Aralkylgruppe mit 7 bis 9 Kohlenstoffatomen darstellt, mittels katalytischer Hydrierung in eine entsprechende Verbindung der

allgemeinen Formel (Ia), in der $R_{1a}$ ein Wasserstoffatom darstellt, oder mittels Raney-Nickel in Gegenwart eines entsprechenden Alkohols in eine entsprechende Verbindung der allgemeinen Formel (Ia), in der $R_{1a}$ eine Alkylgruppe mit 1 bis 12 Kohlenstoffatomen darstellt, übergeführt wird oder
eine erhaltene Verbindung der allgemeinen Formel (Ia), in der $R_{1a}$ und/oder $R_2$ eine Alkoxycarbonylgruppe mit insgesamt 2 bis 4 Kohlenstoffatomen darstellt, mittels Hydrolyse unter gleichzeitiger Decarboxylierung, falls $R_{1a}$ eine Alkoxycarbonylgruppe mit insgesamt 2 bis 4 Kohlenstoffatomen darstellt, in eine entsprechende Verbindung der allgemeinen Formel (Ia), in der $R_{1a}$ ein Wasserstoffatom und/oder $R_2$ eine Carboxygruppe darstellt, übergeführt wird oder
eine erhaltene Verbindung der allgemeinen Formel (Ia) in ihre Salze, insbesondere in ihre physiologisch verträglichen Salze mit einer anorganischen oder organischen Säure oder Base übergeführt wird.

Priorität : 18.02.1981 (DE-31 05 858)

5. Verfahren zur Herstellung von Verbindungen der allgemeinen Formel (Ia), gemäß Anspruch 4, in der $R_{1a}$ mit Ausnahme der Alkoxycarbonylgruppe wie im Anspruch 4 definiert ist, dadurch gekennzeichnet, daß eine gegebenenfalls im Reaktionsgemisch gebildete Carbonsäure der allgemeinen Formel (IIIa)

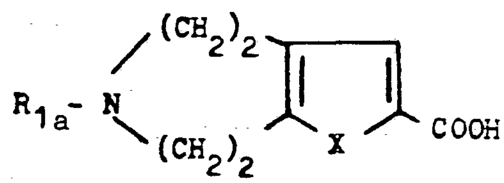

(IIIa)

in der $R_{1a}$ wie im Anspruch 4 definiert ist, decarboxyliert wird
und gewünschtenfalls anschließend eine erhaltene Verbindung der allgemeinen Formel (Ia), in der $R_{1a}$ eine gegebenenfalls im aromatischen Kern durch ein Halogenatom substituierte Aralkylgruppe mit 7 bis 9 Kohlenstoffatomen darstellt, mittels katalytischer Hydrierung in eine entsprechende Verbindung der allgemeinen Formel (Ia), in der $R_{1a}$ ein Wasserstoffatom darstellt, oder mittels Raney-Nickel in Gegenwart eines entsprechenden Alkohols in eine entsprechende Verbindung der allgemeinen Formel (Ia), in der $R_{1a}$ eine Alkylgruppe mit 1 bis 12 Kohlenstoffatomen darstellt, übergeführt wird oder
eine erhaltene Verbindung der allgemeinen Formel (Ia), in der $R_{1a}$ und/oder $R_2$ eine Alkoxycarbonylgruppe mit insgesamt 2 bis 4 Kohlenstoffatomen darstellt, mittels Hydrolyse unter gleichzeitiger Decarboxylierung, falls $R_{1a}$ eine Alkoxycarbonylgruppe mit insgesamt 2 bis 4 Kohlenstoffatomen darstellt, in eine entsprechende Verbindung der allgemeinen Formel (Ia), in der $R_{1a}$ ein Wasserstoffatom und/oder $R_2$ eine Carboxygruppe darstellt, übergeführt wird oder
eine erhaltene Verbindung der allgemeinen Formel (Ia) in ihre Salze, insbesondere in ihre physiologisch verträglichen Salze mit einer anorganischen oder organischen Säure übergeführt wird.

Priorität : 18.02.1981 (DE-31 05 858)

6. Verfahren zur Herstellung von Verbindungen der allgemeinen Formel (Ia) gemäß Anspruch 4, in der $R_{1a}$ mit Ausnahme des Wasserstoffatoms wie im Anspruch 4 definiert ist, dadurch gekennzeichnet, daß eine Verbindung der allgemeinen Formel IV

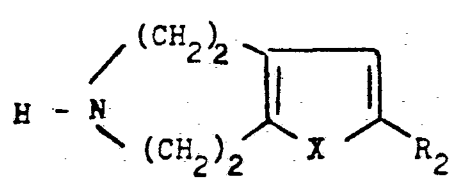

(IV)

in der $R_2$ wie im Anspruch 1 definiert ist, mit einer Verbindung der allgemeinen Formel (Va)

$$R_{1a}—Z \quad \text{(Va)}$$

in der
$R_{1a}$ mit Ausnahme des Wasserstoffatoms wie im Anspruch 4 und Z wie im Anspruch 1 definiert ist, umgesetzt wird
und gewünschtenfalls anschließend eine erhaltene Verbindung der allgemeinen Formel (Ia), in der eine gegebenenfalls im aromatischen Kern durch ein Halogenatom substituierte Aralkylgruppe mit 7 bis 9 Kohlenstoffatomen darstellt, mittels Raney-Nickel in Gegenwart eines entsprechenden Alkohols in eine entsprechende Verbindung der allgemeinen Formel (Ia), in der $R_{1a}$ eine Alkylgruppe mit 1 bis 12 Kohlenstoffatomen darstellt, übergeführt wird oder
eine erhaltene Verbindung der allgemeinen Formel Ia, in der $R_2$ eine Alkoxycarbonylgruppe mit insgesamt 2 bis 4 Kohlenstoffatomen darstellt, mittels Hydrolyse unter in eine entsprechende Verbindung der allgemeinen Formel (Ia), in der $R_2$ eine Carboxygruppe darstellt, übergeführt wird oder

eine erhaltene Verbindung der allgemeinen Formel (Ia) in ihre Salze, insbesondere in ihre physiologisch verträglichen Salze mit einer anorganischen oder organischen Säure oder Base übergeführt wird.

Priorität : 18.02.1981 (DE-31 05 858)

7. Verfahren gemäß den Ansprüchen 1 bis 6, dadurch gekennzeichnet, daß die Umsetzung in einem Lösungsmittel durchgeführt wird.

8. Verfahren gemäß den Ansprüchen 1, 4 und 7, dadurch gekennzeichnet, daß die Umsetzung in Gegenwart einer Base und bei Temperaturen zwischen 0 und 100 °C, vorzugsweise jedoch bei Temperaturen zwischen 0 °C und der Sidetemperatur des Reaktionsgemisches, durchgeführt wird.

9. Verfahren gemäß den Ansprüchen 2, 5 und 7, dadurch gekennzeichnet, daß die Umsetzung in Gegenwart einer Säure und bei erhöhten Temperaturen, vorzugsweise bei der Siedetemperatur des Reaktionsgemisches, z. B. bei Temperaturen zwischen 80 und 100 °C, durchgeführt wird.

10. Verfahren gemäß den Ansprüchen 3, 6 und 7, dadurch gekennzeichnet, daß die Umsetzung falls Z eine nukleophile Austrittsgruppe darstellt, in Gegenwart einer Base und bei Temperaturen zwischen 0 und 150 °C, vorzugsweise jedoch bei Temperaturen zwischen 20 und 80 °C, oder, falls Z eine Hydroxygruppe darstellt, unter Stickstoff und in Gegenwart eines Hydrierungskatalysators bei Temperaturen zwischen 80 und 250 °C, vorzugsweise bei Temperaturen zwischen 100 °C und der Siedetemperatur des eingesetzten Alkohols, oder, falls Z zusammen mit einem Wasserstoffatom des benachbarten Kohlenstoffatoms ein Sauerstoffatom darstellt, mit katalytisch angeregtem Wasserstoff bei Temperaturen zwischen 80 und 250 °C, vorzugsweise jedoch bei Temperaturen zwischen 100 und 200 °C, durchgeführt wird.

**Claims** (for the Contracting States : BE, CH, DE, FR, IT, LI, LU, NL, SE)

1. Azepine derivatives of general formula (I)

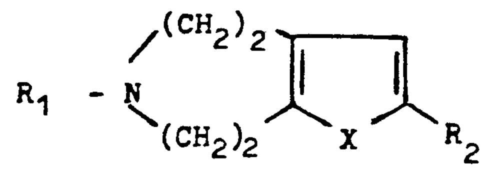

(I)

wherein

$R_1$ represents a hydrogen atom, an alkyl group with 1 to 12 carbon atoms, an allyl group, an aralkyl group with 7 to 9 carbon atoms optionally mono- or disubstituted in the aromatic nucleus by hydroxy groups and/or halogen atoms, a benzoyl group optionally mono- or disubstituted by halogen atoms and/or alkoxy groups with 1 to 3 carbon atoms, an alkoxycarbonyl group with a total of 2 to 4 carbon atoms or an aralkoxycarbonyl group with a total of 8 to 10 carbon atoms,

$R_2$ represents a hydrogen atom, a carboxy group or an alkoxycarbonyl group with a total of 2 to 4 carbon atoms and

X represents an oxygen or sulphur atom or an imino group of formula

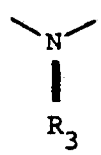

wherein $R_3$ represents a hydrogen atom, an alkyl group with 1 to 3 carbon atoms optionally substituted by a phenyl group, or a phenyl group and the salts thereof with inorganic or organic acids or bases, if $R_2$ represents a carboxy group, particularly the physiologically acceptable salts thereof.

2. Compounds of general formula I as claimed in claim 1, wherein X represents an oxygen or sulphur atom, an imino, methylimino, phenylimino or benzylimino group,

$R_1$ represents a hydrogen atom, an alkyl group with 1 to 3 carbon atoms, a benzyl group optionally mono- or disubstituted in the phenyl nucleus by chlorine or bromine atoms, a benzyl group substituted in the phenyl nucleus by a hydroxy group and a chlorine or bromine atom, a benzoyl group optionally substituted by a chlorine or bromine atom, an alkoxycarbonyl group with a total of 2 to 4 carbon atoms, an allyl, dodecyl, chloromethoxybenzoyl or benzyloxycarbonyl group,

$R_2$ represents a hydrogen atom, a carboxy group or an alkoxycarbonyl group with a total of 2 to 4 carbon atoms, and the salts thereof with inorganic or organic acids or bases if $R_2$ represents a carboxy group, particularly the physiologically acceptable salts thereof.

3. Compounds of general formula I as claimed in claim 1, wherein
X represents a sulphur atom,
$R_1$ represents a dodecyl, benzyl, chlorobenzyl or chlorobenzoyl group and
$R_2$ represents a hydrogen atom or a carboxy group, and the physiologically acceptable salts thereof with inorganic or organic acids or bases.

4. 6-(2-Chlorobenzyl)-5,6,7,8-tetrahydro-4H-thieno [2,3d] azepine and the physiologically acceptable acid addition salts thereof.

5. 6-Benyl-5,6,7,8-tetrahydro-4H-thieno [2,3-d] azepine-2-carboxylic acid and the physiologically acceptable salts thereof.

6. Pharmaceutical compositions containing a compound as claimed in claims 3 to 5 or a physicologically acceptable salt thereof in addition to one or more inert carriers and/or diluents.

7. Compounds as claimed in claims 3 to 5 or the physiologically acceptable salts thereof for use in combating thromboembolic diseases and diseases of the intermediary metabolism.

8. Process for preparing compounds as claimed in claims 1 to 5, characterised in that a compound of general formula II

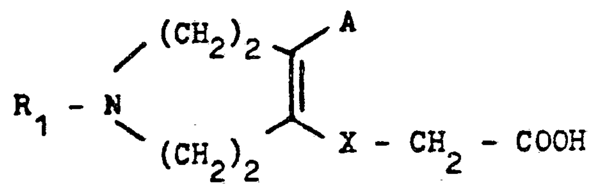

(II)

optionally formed in the reaction mixture,
wherein
$R_1$ and X are defined as in claims 1 to 5 and
A represents a formyl group or the acetals thereof, or an ester thereof is cyclised and optionally the resulting compound is subsequently hydrolysed and/or decarboxylated
and subsequently, if desired, a compound of general formula I obtained wherein $R_1$ represents a benzyl group optionally mono- or disubstituted in the aromatic nucleus by hydroxy groups and/or halogen atoms and/or X represents a benzylimino group, is converted by catalytic hydrogenation into a corresponding compound of general formula I wherein $R_1$ represents a hydrogen atom and/or X represents an imino group, or a compound of general formula I obtained wherein $R_1$ represents a benzyl group optionally mono- or disubstituted in the aromatic nucleus by hydroxy groups and/or halogen atoms is converted by means of Raney nickel in the presence of an appropriate alcohol into a corresponding compound of general formula I wherein $R_1$ represents an alkyl group with 1 to 12 carbon atoms, or
a compound of general formula I obtained wherein $R_1$ and/or $R_2$ represent an alkoxycarbonyl group with a total of 2 to 4 carbon atoms is converted by hydrolysis with simultaneous decarboxylation, if $R_1$ represents an alkoxycarbonyl group with a total of 2 to 4 carbon atoms, into a corresponding compound of general formula I wherein $R_1$ represents a hydrogen atom and/or $R_2$ represents a carboxy group, or a compound of general formula I obtained is converted into the salts thereof, particularly the physiologically acceptable salts thereof with an inorganic or organic acid or base.

9. Process for preparing compounds as claimed in claims 1 to 5 wherein $R_2$ represents a hydrogen atom and $R_1$ has the meanings given in claims 1 to 5, with the exception of the alkoxycarbonyl group, characterised in that a carboxylic acid of general formula III

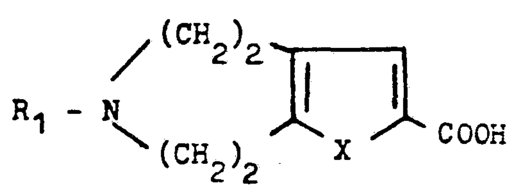

(III)

optionally formed in the reaction mixture, wherein $R_1$ and X are defined as in claims 1 to 5, is decarboxylated
and subsequently, if desired, a compound of general formula I obtained wherein $R_1$ represents a benzyl group optionally mono- or disubstituted in the aromatic nucleus by hydroxy groups and/or halogen atoms and/or X represents a benzylimino group, is converted by catalytic hydrogenation into a corresponding compound of general formula I wherein $R_1$ represents a hydrogen atom and/or X represents an imino group, or a compound of general formula I obtained wherein $R_1$ represents a benzyl group optionally mono- or disubstituted in the aromatic nucleus by hydroxy groups and/or halogen atoms is converted by means of Raney nickel in the presence of an appropriate alcohol into a corresponding compound of general formula I wherein $R_1$ represents an alkyl group with 1 to 12 carbon atoms, or

a compound of general formula I obtained wherein $R_1$ and/or $R_2$ represent an alkoxycarbonyl group with a total of 2 to 4 carbon atoms is converted by hydrolysis with simultaneous decarboxylation, if $R_1$ represents an alkoxycarbonyl group with a total of 2 to 4 carbon atoms, into a corresponding compound of general formula I wherein $R_1$ represents a hydrogen atom and/or $R_2$ represents a carboxy group, or a compound of general formula I obtained is converted into the salts thereof, particularly the physiologically acceptable salts thereof with an inorganic or organic acid or base.

10. Process for preparing compounds as claimed in claims 1 to 5 wherein $R_1$ has the meanings given in claims 1 to 5 with the exception of a hydrogen atom, characterised in that a compound of general formula IV

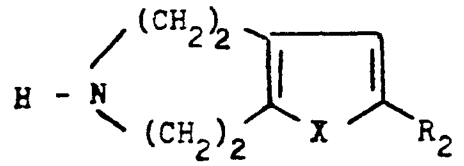

(IV)

wherein $R_2$ and X are defined as in claims 1 to 5 is reacted with a compound of general formula V

$$R_1{-}Z \qquad (V)$$

wherein

$R_1$ has the definitions given in claims 1 to 5, with the exception of the hydrogen atom, and

Z represents a nucleophilic leaving group, a hydroxy group or together with a hydrogen atom of the adjacent carbon atom represents an oxygen atom,

and subsequently, if desired, a compound of general formula I obtained wherein X represents a benzylimino group is converted by catalytic hydrogenation into a corresponding compound of general formula I wherein X represents an imino group, or a compound of general formula I obtained wherein $R_1$ represents a benzyl group optionally mono- or disubstituted in the aromatic nucleus by hydroxy groups and/or halgoen atoms is converted by means of Raney nickel in the presence of a corresponding alcohol into a corresponding compound of general formula I wherein $R_1$ represents an alkyl group with 1 to 12 carbon atoms, or

a compound of general formula I obtained wherein $R_2$ represents an alkoxycarbonyl group with a total of 2 to 4 carbon atoms is converted by hydrolysis into a corresponding compound of general formula I wherein $R_2$ represents a carboxy group, or

a compound of general formula I is converted into the salts thereof, particularly the physiologically acceptable salts thereof with an inorganic or organic acid or base.

**Claims** (for the Contracting State AT)

1. Process for preparing azepine derivatives of general formula I

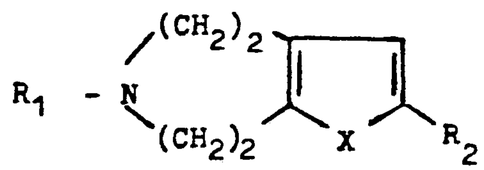

(I)

wherein

$R_1$ represents a hydrogen atom, an alkyl group with 1 to 12 carbon atoms, an allyl group, an aralkyl group with 7 to 9 carbon atoms optionally mono- or disubstituted in the aromatic nucleus by hydroxy groups and/or halogen atoms, a benzoyl group optionally mono- or disubstituted by halogen atoms and/or alkoxy groups with 1 to 3 carbon atoms, an alkoxycarbonyl group with a total of 2 to 4 carbon atoms or an aralkoxycarbonyl group with a total of 8 to 10 carbon atoms,

$R_2$ represents a hydrogen atom, a carboxy group or an alkoxycarbonyl group with a total of 2 to 4 carbon atoms and

X represents an oxygen or sulphur atom or an imino group of formula

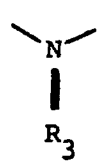

wherein $R_3$ represents a hydrogen atom, an alkyl group with 1 to 3 carbon atoms optionally substituted by a phenyl group, or a phenyl group and the salts thereof with inorganic or organic acids or bases, if $R_2$ represents a carboxy group, particularly the physiologically acceptable salts thereof, characterised in that a compound of general formula II

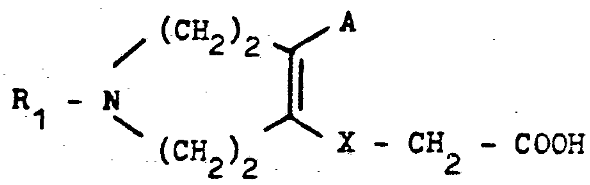

(II)

optionally formed in the reaction mixture,
wherein
$R_1$ and X are as hereinbefore defined and
A represents a formyl group or the acetals thereof, or an ester thereof is cyclised and optionally the resulting compound is subsequently hydrolysed and/or decarboxylated
and subsequently, if desired, a compound of general formula I obtained wherein $R_1$ represents a benzyl group optionally mono- or disubstituted in the aromatic nucleus by hydroxy groups and/or halogen atoms and/or X represents a benzylimino group, is converted by catalytic hydrogenation into a corresponding compound of general formula I wherein $R_1$ represents a hydrogen atom and/or X represents an imino group, or a compound of general formula I obtained wherein $R_1$ represents a benzyl group optionally mono- or disubstituted in the aromatic nucleus by hydroxy groups and/or halogen atoms is converted by means of Raney nickel in the presence of an appropriate alcohol into a corresponding compound of general formula I wherein $R_1$ represents an alkyl group with 1 to 12 carbon atoms, or
a compound of general formula I obtained wherein $R_1$ and/or $R_2$ represent an alkoxycarbonyl group with a total of 2 to 4 carbon atoms is converted by hydrolysis with simultaneous decarboxylation, if $R_1$ represents an alkoxycarbonyl group with a total of 2 to 4 carbon atoms, into a corresponding compound of general formula I wherein $R_1$ represents a hydrogen atom and/or $R_2$ represents a carboxy group, or a compound of general formula I obtained is converted into the salts thereof, particularly the physiologically acceptable salts thereof with an inorganic or organic acid or base.

2. Process for the preparation of compounds of general formula I as claimed in claim 1 wherein
$R_2$ represents a hydrogen atom and
$R_1$ has the meanings given in claim 1,
with the exception of the alkoxycarbonyl group, characterised in that a carboxylic acid of general formula III

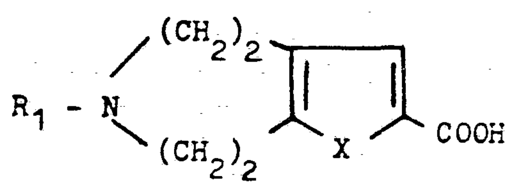

(III)

optionally formed in the reaction mixture,
wherein $R_1$ and X are defined as in claim 1, is decarboxylated
and subsequently, if desired, a compound of general formula I obtained wherein $R_1$ represents a benzyl group optionally mono- or disubstituted in the aromatic nucleus by hydroxy groups and/or halogen atoms and/or X represents a benzylimino group, is converted by catalytic hydrogenation into a corresponding compound of general formula I wherein $R_1$ represents a hydrogen atom and/or X represents an imino group, or a compound of general formula I obtained wherein $R_1$ represents a benzyl group optionally mono- or disubstituted in the aromatic nucleus by hydroxy groups and/or halogen atoms is converted by means of Raney nickel in the presence of an appropriate alcohol into a corresponding compound of general formula I wherein $R_1$ represents an alkyl group with 1 to 12 carbon atoms, or
a compound of general formula I obtained wherein $R_1$ and/or $R_2$ represent an alkoxycarbonyl group with a total of 2 to 4 carbon atoms is converted by hydrolysis with simultaneous decarboxylation, if $R_1$ represents an alkoxycarbonyl group with a total of 2 to 4 carbon atoms, into a corresponding compound of general formula I wherein $R_1$ represents a hydrogen atom and/or $R_2$ represents a carboxy group, or a compound of general formula I obtained is converted into the salts thereof, particularly the physiologically acceptable salts thereof with an inorganic or organic acid or base.

3. Process for the preparation of compounds of general formula I as claimed in claim 1 wherein $R_1$ has the meanings given in claim 1 with the exception of a hydrogen atom, characterised in that a compound of general formula IV

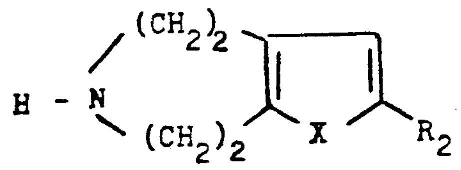

(IV)

wherein $R_2$ and X are defined as in claim 1 is reacted with a compound of general formula V

$$R_1—Z \quad (V)$$

wherein

$R_1$ has the meanings given in claim 1, with the exception of the hydrogen atom, and

Z represents a nucleophilic leaving group, a hydroxy group or together with a hydrogen atom of the adjacent carbon atom represents an oxygen atom,

and subsequently, if desired, a compound of general formula I obtained wherein X represents a benzylimino group is converted by catalytic hydrogenation into a corresponding compound of general formula I wherein X represents an imino group, or a compound of general formula I obtained wherein $R_1$ represents a benzyl group optionally mono- or disubstituted in the aromatic nucleus by hydroxy groups and/or halogen atoms is converted by means of Raney nickel in the presence of a corresponding alcohol into a corresponding compound of general formula I wherein $R_1$ represents an alkyl group with 1 to 12 carbon atoms, or

a compound of general formula I obtained wherein $R_2$ represents an alkoxycarbonyl group with a total of 2 to 4 carbon atoms is converted by hydrolysis into a corresponding compound of general formula I wherein $R_2$ represents a carboxy group, or

a compound of general formula I is converted into the salts thereof, particularly the physiologically acceptable salts thereof with an inorganic or organic acid or base.

4. Process for the preparation of compounds of general formula Ia

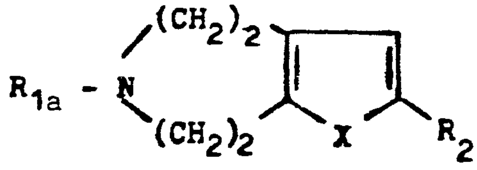

(Ia)

wherein

$R_2$ and X are as defined in claim 1 and

$R_{1a}$ represents a hydrogen atom, an alkyl group with 1 to 12 carbon atoms, an allyl group, an aralkyl group with 7 to 9 carbon atoms optionally substituted by a halogen atom in the aromatic nucleus, a benzoyl group optionally mono- or disubstituted by a halogen atom and/or an alkoxy group with 1 to 3 carbon atoms, an alkoxycarbonyl group with a total of 2 to 4 carbon atoms or an aralkoxycarbonyl group with a total of 8 to 10 carbon atoms, characterised in that a compound of general formula IIa

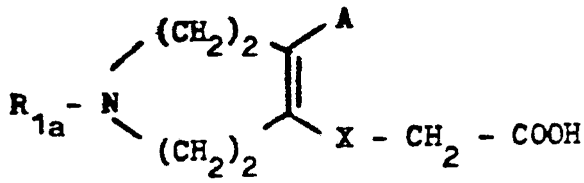

(IIa)

optionally formed in the reaction mixture, wherein $R_{1a}$ as hereinbefore defined, or an ester thereof, is cyclised and optionally the resulting compound is subsequently hydrolysed and/or decarboxylated

and subsequently, if desired, a compound of general formula Ia obtained wherein $R_{1a}$ represents an aralkyl group with 7 to 9 carbon atoms optionally substituted by a halogen atom in the aromatic nucleus is converted by catalytic hydrogenation to a corresponding compound of general formula Ia wherein $R_{1a}$ represents a hydrogen atom or is converted by Raney nickel in the presence of a corresponding alcohol into a corresponding compound formula Ia wherein $R_{1a}$ represents an alkyl group with 1 to 12 carbon atoms, or

a compound of general formula Ia obtained wherein $R_{1a}$ and/or $R_2$ represents an alkoxycarbonyl group with a total of 2 to 4 carbon atoms is converted by hydroxylis with simultaneous decarboxylation, if $R_{1a}$ represents an alkoxycarbonyl group with a total of 2 to 4 carbon atoms, into a corresponding compound

of general formula Ia wherein $R_{1a}$ represents a hydrogen atom and/or $R_2$ represents a carboxy group, or a compound of general formula Ia obtained is converted into the salts thereof, particularly the physiologically acceptable salts thereof with an inorganic or organic acid or base.

Priority : 18.2.1981 (DE-31 05 858)

5. Process for preparing compounds of general formula Ia as claimed in claim 4 wherein $R_{1a}$ has the meanings given in claim 4 with the exception of the alkoxycarbonyl group, characterised in that a carboxylic acid of general formula IIIa

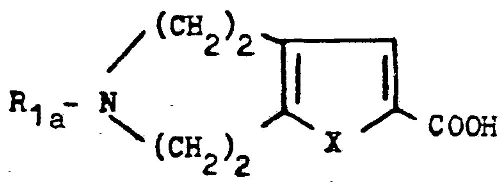

(IIIa)

optionally formed in the reaction mixture,
wherein $R_{1a}$ is defined as in claim 4, is decarboxylated
and subsequently, if desired, a compound of general formula Ia obtained wherein $R_{1a}$ represents an aralkyl group with 7 to 9 carbon atoms optionally substituted by a halogen atom in the aromatic nucleus is converted by catalytic hydrogenation to a corresponding compound of general formula Ia wherein $R_{1a}$ represents a hydrogen atom or is converted by Raney nickel in the presence of a corresponding alcohol into a corresponding compound of general formula Ia wherein $R_{1a}$ represents an alkyl group with 1 to 12 carbon atoms, or
a compound of general formula Ia obtained wherein $R_{1a}$ and/or $R_2$ represents an alkoxycarbonyl group with a total of 2 to 4 carbon atoms is converted by hydrolysis with simultaneous decarboxylation, if $R_{1a}$ represents an alkoxycarbonyl group with a total of 2 to 4 carbon atoms, into a corresponding compound of general formula Ia wherein $R_{1a}$ represents a hydrogen atom and/or $R_2$ represents a carboxy group, or a compound of general formula Ia obtained is converted into the salts thereof, particularly the physiologically acceptable salts thereof with an inorganic or organic acid or base.

Priority : 18.02.1981 (DE-31 05 858)

6. Process for preparing compounds of general formula Ia as claimed in claim 4 wherein $R_{1a}$ has the meanings given in claim 4 with the exception of the hydrogen atom, characterised in that a compound of general formula IV

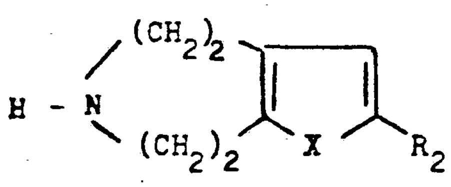

(IV)

wherein $R_2$ is defined as in claim 1 is reacted with a compound of general formula Va

$$R_{1a}—Z \qquad \text{(Va)}$$

wherein
$R_{1a}$ has the meanings given in claim 4 with the exception of the hydrogen atom and
Z is defined as in claim 1,
and subsequently if desired a compound of general formula Ia obtained wherein $R_{1a}$ represents an aralkyl group with 7 to 9 carbon atoms optionally substituted by a halogen atom in the aromatic nucleus is converted by means of Raney nickel in the presence of a corresponding alcohol into a corresponding compound of general formula Ia wherein $R_{1a}$ represents an alkyl group with 1 to 12 carbon atoms, or
a compound of general formula Ia obtained wherein $R_2$ represents an alkoxycarbonyl group with a total of 2 to 4 carbon atoms is converted by hydrolysis into a corresponding compound of general formula Ia wherein $R_2$ represents a carboxy group, or
a compound of general formula Ia obtained is converted into the salts thereof, particularly the physiologically acceptable salts thereof with an inorganic or organic acid or base.

Priority : 18.02.1981 (DE-31 05 858)

7. Process as claimed in claims 1 to 6, characterised in that the reaction is carried out in a solvent.

8. Process as claimed in claims 1, 4 and 7, characterised in that the reaction is carried out in the presence of a base and at temperatures of between 0 and 100 °C but preferably at temperatures of between 0 °C and the boiling temperature of the reaction mixture.

9. Process as claimed in claims 2, 5 and 7, characterised in that the reaction is carried out in the presence of an acid and at elevated temperatures, preferably at the boiling temperature of the reaction mixture, e. g. at temperatures of between 80 and 100 °C.

10. Process as claimed in claims 3, 6 and 7, characterised in that if Z represents a nucleophilic leaving group the reaction is carried out in the presence of a base and at temperatures of between 0 and 150 °C, preferably at temperatures of between 20 and 80 °C or, if Z represents a hydroxy group, it is carried out in a nitrogen atmosphere and in the presence of a hydrogenation catalyst at temperatures of between 80 and 250 °C preferably at temperatures of between 100 °C and the boiling temperature of the alcohol used or, if Z together with a hydrogen atom of the adjacent carbon atom represents an oxygen atom, the reaction is carried out with catalytically activated hydrogen at temperatures of between 80 and 250 °C, preferably at temperatures of between 100 and 200 °C.

**Revendications** (pour les Etats contractants : BE, CH, DE, FR, IT, LI, LU, NL, SE)

1. Dérivés de l'azépine de formule générale (I)

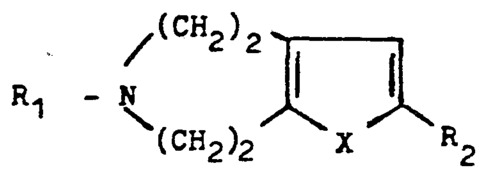

(I)

dans laquelle

$R_1$ représente un atome d'hydrogène, un groupe alcoyle avec 1 à 12 atomes de carbone, un groupe allyle, un groupe aralcoyle avec 7 à 9 atomes de carbone, éventuellement mono ou disubstitué dans le noyau aromatique par des groupes hydroxy et/ou des atomes d'halogène, ou représente un groupe benzoyle éventuellement mono- ou disubstitué par des atomes d'halogène et/ou des groupes alcoxy avec 1 à 3 atomes de carbone, ou représente un groupe alcoxycarboxyle avec en tout 2 à 4 atomes de carbone ou un groupe aralcoxycarbonyle avec en tout 8 à 10 atomes de carbone,

$R_2$ représente un atome d'hydrogène, un groupe carboxy ou un groupe alcoxycarbonyle avec en tout 2 à 4 atomes de carbone et

X représente un atome d'oxygène ou de soufre ou un groupe imino de formule

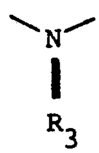

dans laquelle $R_3$ représente un atome d'hydrogène, un groupe alcoyle avec 1 à 3 atomes de carbone, éventuellement substitué par un groupe phényle ou représente un groupe phényle, et leurs sels avec des acides minéraux ou organiques ou des bases, lorsque $R_2$ représente un groupe carboxy, en particulier leurs sels physiologiquement supportables.

2. Composés de formule générale (I) selon la revendication 1, dans laquelle

X représente un atome d'oxygène ou de soufre, un groupe imino, méthylimino, phénylimino ou benzylimino,

$R_1$ représente un atome d'hydrogène, un groupe alcoyle avec 1 à 3 atomes de carbone, un groupe benzyle éventuellement mono- ou disubstitué dans le noyau phényle par des atomes de chlore ou de brome, ou représente un groupe benzyle substitué dans le noyau phényle par un groupe hydroxy et un atome de chlore ou de brome, ou représente un groupe benzoyle éventuellement substitué par un atome de chlore ou de brome, ou représente un groupe alcoxycarbonyle avec en tout 2 à 4 atomes de carbone, un groupe allyle, dodécyle, chloro-méthoxy-benzoyle ou benzyloxycarbonyle,

$R_2$ représente un atome d'hydrogène, un groupe carboxy ou un groupe alcoxycarbonyle avec en tout 2 à 4 atomes de carbone, et leurs sels avec des acides minéraux ou organiques ou des bases, lorsque $R_2$ représente un groupe carboxy, en particulier leurs sels physiologiquement supportables.

3. Composés de formule générale (I) selon la revendication 1, dans laquelle

X représente un atome de soufre,

$R_1$ représente un groupe dodécyle, benzyle, chlorobenzyle ou chlorobenzoyle et

$R_2$ représente un atome d'hydrogène ou le groupe carboxy, et leurs sels physiologiquement supportables avec des acides ou des bases minéraux ou organiques.

4. La 6-(2-chlorobenzyl)-5,6,7,8-tétrahydro-4H-thiéno [2,3-d] azépine et ses sels d'addition d'acides physiologiquement supportables.

5. L'acide 6-benzyl-5,6,7,8-tétrahydro-4H-thiéno [2,3-d] azépine-2-carboxylique et ses sels physiologiquement supportables.

6. Médicament contenant un composé selon les revendications 3 à 5 ou son sel physiologiquement supportable conjointement à un ou plusieurs excipients et/ou diluants inertes.

7. Composés selon les revendications 3 à 5 ou leurs sels physiologiquement supportables pour

l'utilisation dans la lutte contre les maladies thromboemboliques et les maladies du métabolisme intermédiaire.

8. Procédé pour la préparation de composés selon les revendications 1 à 5, caractérisé en ce qu'on cyclise un composé éventuellement formé dans le mélange réactionnel, de formule générale (II)

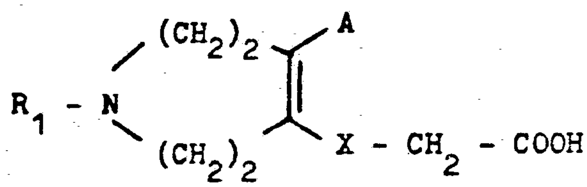

(II)

dans laquelle

$R_1$ et X sont définis comme dans les revendications 1 à 5 et

A représente le groupe formyle ou ses acétals, ou son ester et en ce qu'on hydrolyse et/ou décarboxyle éventuellement ensuite le composé obtenu,

et si on le désire ensuite, on transforme un composé obtenu de formule générale (I) dans laquelle $R_1$ représente un groupe benzyle éventuellement mono- ou disubstitué dans le noyau aromatique par des groupes hydroxy et/ou des atomes d'halogène et/ou X représente un groupe benzylimino, au moyen d'une hydrogénation catalytique en un composé correspondant de formule générale (I) dans laquelle $R_1$ représente un atome d'hydrogène et/ou X représente un groupe imino ou on transforme un composé obtenu de formule générale (I), dans laquelle $R_1$ représente un groupe benzyle éventuellement mono- ou disubstitué dans le noyau aromatique par des groupes hydroxy et/ou des atomes d'halogène, au moyen de nickel de Raney en présence d'un alcool correspondant en un composé correspondant de formule générale (I) dans laquelle $R_1$ représente un groupe alcoyle avec 1 à 12 atomes de carbone, ou on transforme un composé obtenu de formule générale (I)

dans laquelle $R_1$ et/ou $R_2$ représentent un groupe alcoxycarbonyle avec en tout 2 à 4 atomes de carbone, au moyen d'une hydrolyse avec décarboxylation simultanée, dans le cas où $R_1$ représente un groupe alcoxycarbonyle avec en tout 2 à 4 atomes de carbone, en un composé correspondant de formule générale (I) dans laquelle $R_1$ représente un atome d'hydrogène et/ou $R_2$ représente un groupe carboxy ou on transforme un composé obtenu de formule générale (I) en ses sels, en particulier en ses sels physiologiquement supportables avec des acides ou des bases minéraux ou organiques.

9. Procédé pour la préparation de composés selon les revendications 1 à 5, dans lesquels $R_2$ représente un atome d'hydrogène et $R_1$ a les significations indiquées dans les revendications 1 à 5 à l'exception du groupe alcoxycarbonyle, caractérisé en ce qu'on décarboxyle un acide carboxylique éventuellement formé dans le mélange réactionnel, de formule générale (III)

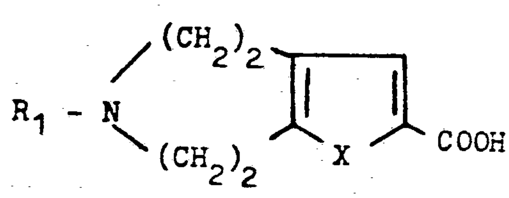

(III)

dans laquelle $R_1$ et X sont définis comme dans les revendications 1 à 5, et si on le désire ensuite, on transforme un composé obtenu de formule générale I dans laquelle $R_1$ représente un groupe benzyle éventuellement mono- ou disubstitué dans le noyau aromatique par des groupes hydroxy et/ou des atomes d'halogène et/ou X représente un groupe benzylimino, au moyen d'une hydrogénation catalytique en un composé correspondant de formule générale (I), dans laquelle $R_1$ représente un atome d'hydrogène et/ou X représente un groupe imino ou on transforme un composé obtenu de formule générale (I) dans laquelle $R_1$ représente un groupe benzyle éventuellement mono- ou disubstitué dans le noyau aromatique par des groupes hydroxy et/ou des atomes d'halogène, au moyen de nickel de Raney en présence d'un alcool correspondant en un composé correspondant de formule générale (I) dans laquelle $R_1$ représente un groupe alcoyle avec 1 à 12 atomes de carbone ou

on transforme un composé obtenu de formule générale (I) dans laquelle $R_1$ et/ou $R_2$ représentent un groupe alcoxycarbonyle avec en tout 2 à 4 atomes de carbone, au moyen d'une hydrolyse avec décarboxylation simultanée, dans le cas où $R_1$ représente un groupe alcoxycarbonyle avec en tout 2 à 4 atomes de carbone, en un composé correspondant de formule générale (I) dans laquelle $R_1$ représente un atome d'hydrogène et/ou $R_2$ représente un groupe carboxy ou on transforme un composé obtenu de formule générale (I) en ses sels, en particulier en ses sels physiologiquement supportables avec un acide minéral ou organique.

10. Procédé pour la préparation de composés selon les revendications 1 à 5 dans lesquels $R_1$ a les significations indiquées dans les revendications 1 à 5 à l'exception de l'atome d'hydrogène, caractérisé en ce qu'on fait réagir un composé de formule générale (IV)

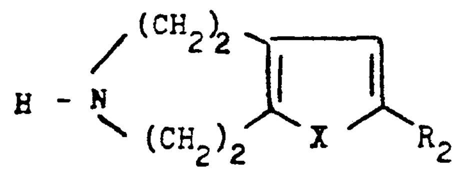

(IV)

dans laquelle $R_2$ et X sont définis comme dans les revendications 1 à 5, avec un composé de formule générale (V)

$R_1$—Z (V)

dans laquelle

$R_1$ est défini comme dans les revendications 1 à 5, à l'exception de l'atome d'hydrogène et

Z représente un groupe partant nucléophile, un groupe hydroxy ou ensemble avec un atome d'hydrogène de l'atome de carbone voisin, un atome d'oxygène

et si on le désire ensuite, on transforme un composé obtenu de formule générale (I) dans laquelle X représente un groupe benzylimino, au moyen d'une hydrogénation catalytique en un composé correspondant de formule générale (I) dans laquelle X représente un groupe imino ou on transforme un composé obtenu de formule générale (I) dans laquelle $R_1$ représente un groupe benzyle éventuellement mono- ou disubstitué dans le noyau aromatique par des groupes hydroxy et/ou des atomes d'halogène, au moyen de nickel de Raney en présence d'un alcool correspondant en un composé correspondant de formule générale (I) dans laquelle $R_1$ représente un groupe alcoyle avec 1 à 12 atomes de carbone ou on transforme un composé obtenu de formule générale (I), dans laquelle $R_2$ représente un groupe alcoxycarbonyle avec en tout 2 à 4 atomes de carbone, au moyen d'une hydrolyse en un composé correspondant de formule générale (I) dans laquelle $R_2$ représente un groupe carboxy ou on transforme un composé obtenu de formule générale (I) en ses sels, en particulier en ses sels physiologiquement supportables avec un acide ou une base minéral(e) ou organique.

**Revendications** (pour l'Etat contractant AT)

1. Procédé pour la préparation de dérivés de l'azépine de formule générale (I)

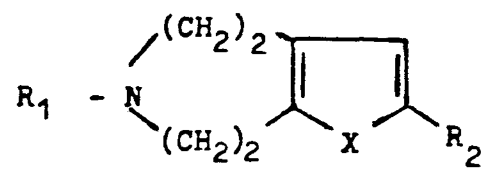

(I)

dans laquelle

$R_1$ représente un atome d'hydrogène, un groupe alcoyle avec 1 à 12 atomes de carbone, un groupe allyle, un groupe aralcoyle avec 7 à 9 atomes de carbone, éventuellement mono ou disubsitué dans le noyau aromatique par des groupes hydroxy et/ou des atomes d'halogène, ou représente un groupe benzoyle éventuellement mono- ou disubstitué par des atomes d'halogène et/ou des groupes alcoxy avec 1 à 3 atomes de carbone, ou représente un groupe alcoxycarbonyle avec en tout 2 à 4 atomes de carbone ou un groupe aralcoxycarbonyle avec en tout 8 à 10 atomes de carbone,

$R_2$ représente un atome d'hydrogène, un groupe carboxy ou un groupe alcoxycarbonyle avec en tout 2 à 4 atomes de carbone et

X représente un atome d'oxygène ou de soufre ou un groupe imino de formule

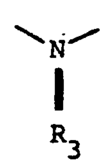

dans laquelle $R_3$ représente un atome d'hydrogène, un groupe alcoyle avec 1 à 3 atomes de carbone, éventuellement substitué par un groupe phényle ou représente un groupe phényle, et de leurs sels avec des acides minéraux ou organiques ou des bases, lorsque $R_2$ représente un groupe carboxy, en particulier de leurs sels physiologiquement supportables, caractérisé en ce qu'on cyclise un composé éventuellement formé dans le mélange réactionnel, de formule générale (II)

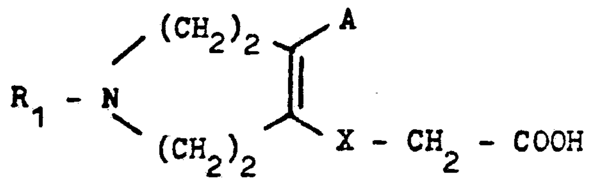

(II)

dans laquelle

$R_1$ et X sont définis au début et

A représente le groupe formyle ou ses acétals, ou son ester et en ce qu'on hydrolyse et/ou décarboxyle éventuellement ensuite le composé obtenu

et si on le désire ensuite, on transforme un composé obtenu de formule générale (I), dans laquelle $R_1$ représente un groupe benzyle éventuellement mono- ou disubstitué dans le noyau aromatique par des groupes hydroxy et/ou des atomes d'halogène et/ou X représente un groupe benzylimino, au moyen d'une hydrogénation catalytique en un composé correspondant de formule générale (I) dans laquelle $R_1$ représente un atome d'hydrogène et/ou X représente un groupe imino ou on transforme un composé obtenu de formule générale (I) dans laquelle $R_1$ représente un groupe benzyle éventuellement mono- ou disubstitué dans le noyau aromatique par des groupes hydroxy et/ou des atomes d'halogène, au moyen de nickel de Raney en présence d'un alcool correspondant en un composé correspondant de formule générale (I) dans laquelle $R_1$ représente un groupe alcoyle avec 1 à 12 atomes de carbone ou on transforme un composé obtenu de formule générale (I) dans laquelle $R_1$ et/ou $R_2$ représentent un groupe alcoxycarbonyle avec en tout 2 à 4 atomes de carbone, au moyen d'une hydrolyse avec décarboxylation simultanée, dans le cas où $R_1$ représente un groupe alcoxycarbonyle avec en tout 2 à 4 atomes de carbone, en un composé correspondant de formule générale (I) dans laquelle $R_1$ représente un atome d'hydrogène et/ou $R_2$ représente un groupe carboxy ou on transforme un composé obtenu de formule générale (I) en ses sels, en particulier en ses sels physiologiquement supportables avec un acide ou une base minéral(e) ou organique.

2. Procédé pour la préparation de composés de formule générale (I) selon la revendication 1, dans laquelle $R_2$ représente un atome d'hydrogène et $R_1$ a les significations indiquées dans la revendication 1 à l'exception du groupe alcoxycarbonyle, caractérisé en ce qu'on décarboxyle un acide carboxylique éventuellement formé dans le mélange réactionnel, de formule générale (III)

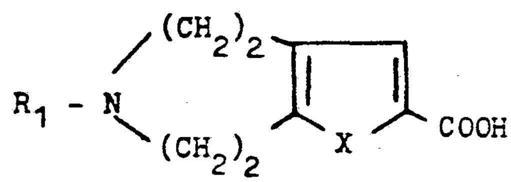

(III)

dans laquelle $R_1$ et X sont définis comme dans la revendication 1,

et en ce que si on le désire ensuite, on transforme un composé obtenu de formule générale (I), dans laquelle $R_1$ représente un groupe benzyle éventuellement mono- ou disubstitué dans le noyau aromatique par des groupes hydroxy et/ou des atomes d'halogène et/ou X représente un groupe benzylimino, au moyen d'une hydrogénation catalytique en un composé correspondant de formule générale (I) dans laquelle $R_1$ représente un atome d'hydrogène et/ou X représente un groupe imino ou on transforme un composé obtenu de formule générale (I) dans laquelle $R_1$ représente un groupe benzyle éventuellement mono- ou disubstitué dans le noyau aromatique par des groupes hydroxy et/ou des atomes d'halogène, au moyen de nickel de Raney en présence d'un alcool correspondant en un composé correspondant de formule générale (I) dans laquelle $R_1$ représente un groupe alcoyle avec 1 à 12 atomes de carbone ou

on transforme un composé obtenu de formule générale (I) dans laquelle $R_1$ et/ou $R_2$ représentent un groupe alcoxycarbonyle avec en tout 2 à 4 atomes de carbone, au moyen d'une hydrolyse avec décarboxylation simultanée, dans le cas où $R_1$ représente un groupe alcoxycarbonyle avec en tout 2 à 4 atomes de carbone, en un composé correspondant de formule générale (I) dans laquelle $R_1$ représente un atome d'hydrogène et/ou $R_2$ représente un groupe carboxy ou on transforme un composé obtenu de formule générale (I) en ses sels, en particulier en ses sels physiologiquement supportables avec des acides minéraux ou organiques.

3. Procédé pour la préparation de composés de formule générale (I) selon la revendication 1, dans laquelle $R_1$ a les significations indiquées dans la revendication 1 à l'exception de l'atome d'hydrogène, caractérisé en ce qu'on fait réagir un composé de formule générale (IV)

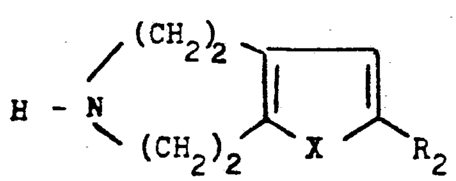

(IV)

dans laquelle $R_2$ et X sont définis comme dans la revendication 1, avec un composé de formule générale (V)

$$R_1—Z \qquad (V)$$

dans laquelle

$R_1$ est défini comme dans la revendication 1 à l'exception de l'atome d'hydrogène et

Z représente un groupe partant nucléophile, un groupe hydroxy ou ensemble avec un atome d'hydrogène de l'atome de carbone voisin, un atome d'oxygène

et si on le désire ensuite, on transforme un composé obtenu de formule générale (I) dans laquelle X représente un groupe benzylimino, au moyen d'une hydrogénation catalytique en un composé correspondant de formule générale (I) dans laquelle X représente un groupe imino ou on transforme un composé obtenu de formule générale (I) dans laquelle $R_1$ représente un groupe benzyle, éventuellement mono- ou disubstitué dans le noyau aromatique par des groupes hydroxy et/ou des atomes d'halogène, au moyen de nickel de Raney en présence d'un alcool correspondant en un composé correspondant de formule générale (I) dans laquelle $R_1$ représente un groupe alcoyle avec 1 à 12 atomes de carbone ou on transforme un composé obtenu de formule générale (I) dans laquelle $R_2$ représente un groupe alcoxycarbonyle avec en tout 2 à 4 atomes de carbone, au moyen d'une hydrolyse en un composé correspondant de formule générale (I) dans laquelle $R_2$ représente un groupe carboxy ou on transforme un composé obtenu de formule générale (I) en ses sels, en particulier en ses sels physiologiquement supportables avec un acide ou une base minéral(e) ou organique.

4. Procédé pour la préparation de composés de formule générale (Ia)

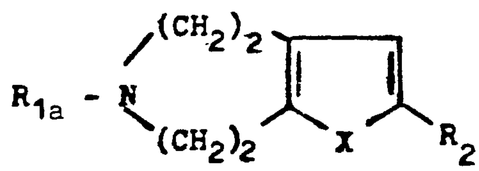

(Ia)

dans laquelle

$R_2$ et X sont définis comme dans la revendication 1 et $R_{1a}$ représente un atome d'hydrogène, un groupe alcoyle avec 1 à 12 atomes de carbone, un groupe allyle, un groupe aralcoyle avec 7 à 9 atomes de carbone, éventuellement substitué dans le noyau aromatique par un atome d'halogène, ou représente un groupe benzoyle éventuellement mono- ou disubstitué par un atome d'halogène et/ou un groupe alcoxy avec 1 à 3 atomes de carbone, ou représente un groupe alcoxycarbonyle avec en tout 2 à 4 atomes de carbone ou un groupe aralcoxycarbonyle avec en tout 8 à 10 atomes de carbone, caractérisé en ce qu'on cyclise un composé éventuellement formé dans le mélange réactionnel, de formule générale (IIa)

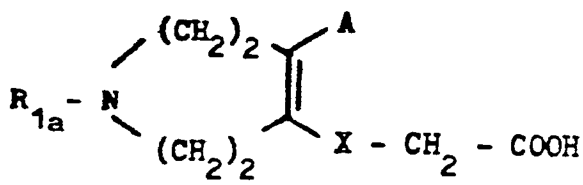

(IIa)

dans laquelle $R_{1a}$ est défini comme au début, ou son ester et en ce qu'éventuellement on hydrolyse et/ou décarboxyle ensuite le composé obtenu

et si on le désire ensuite, on transforme un composé obtenu de formule générale (Ia) dans laquelle $R_{1a}$ représente un groupe aralcoyle avec 7 à 9 atomes de carbone éventuellement substitué dans le noyau aromatique par un atome d'halogène, au moyen d'une hydrogénation catalytique en un composé correspondant de formule générale (Ia), dans laquelle $R_{1a}$ représente un atome d'hydrogène, ou au moyen de nickel de Raney en présence d'un alcool correspondant en un composé correspondant de formule générale (Ia) dans laquelle $R_{1a}$ représente un groupe alcoyle avec 1 à 12 atomes de carbone ou on transforme un composé obtenu de formule générale (Ia) dans laquelle $R_{1a}$ et/ou $R_2$ représentent un groupe alcoxycarbonyle avec en tout 2 à 4 atomes de carbone, au moyen d'une hydrolyse avec décarboxylation simultanée, dans le cas où $R_{1a}$ représente un groupe alcoxycarbonyle avec en tout 2 à 4 atomes de carbone, en un composé correspondant de formule générale (Ia) dans laquelle $R_{1a}$ représente un atome d'hydrogène et/ou $R_2$ représente un groupe carboxy ou on transforme un composé obtenu de formule générale (Ia) en ses sels, en particulier en ses sels physiologiquement supportables avec un acide ou une base minéral(e) ou organique.

Priorité : 18.02.1981 (DE-31 05 858).

5. Procédé pour la préparation de composés de formule générale (Ia) selon la revendication 4, dans

laquelle $R_{1a}$ est défini comme dans la revendication 4 à l'exception du groupe alcoxycarbonyle, caractérisé en ce qu'on décarboxyle un acide carboxylique éventuellement formé dans le mélange réactionnel, de formule générale (IIIa)

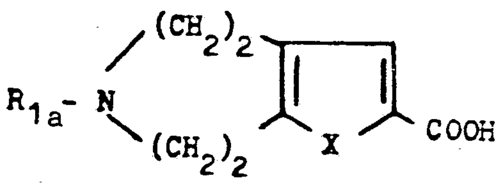

(IIIa)

dans laquelle $R_{1a}$ est défini comme dans la revendication 4
et si on le désire ensuite, on transforme un composé obtenu de formule générale (Ia) dans laquelle $R_{1a}$ représente un groupe aralcoyle avec 7 à 9 atomes de carbone, éventuellement substitué dans le noyau aromatique par un atome d'halogène, au moyen d'une hydrogénation catalytique en un composé correspondant de formule générale (Ia) dans laquelle $R_{1a}$ représente un atome d'hydrogène, ou au moyen de nickel de Raney en présence d'un alcool correspondant en un composé correspondant de formule générale (Ia) dans laquelle $R_{1a}$ représente un groupe alcoyle avec 1 à 12 atomes de carbone ou on transforme un composé obtenu de formule générale (Ia) dans laquelle $R_{1a}$ et/ou $R_2$ représentent un groupe alcoxycarbonyle avec en tout 2 à 4 atomes de carbone, au moyen d'une hydrolyse avec décarboxylation simultanée, dans le cas où $R_{1a}$ représente un groupe alcoxycarbonyle avec en tout 2 à 4 atomes de carbone, en un composé correspondant de formule générale (Ia) dans laquelle $R_{1a}$ représente un atome d'hydrogène et/ou $R_2$ représente un groupe carboxy ou
on transforme un composé obtenu de formule générale (Ia) en ses sels, en particulier en ses sels physiologiquement supportables avec un acide minéral ou organique.
Priorité : 18.02.1981 (DE-31 05 858)

6. Procédé pour la préparation de composés de formule générale (Ia) selon la revendication 4, dans laquelle $R_{1a}$ est défini comme dans la revendication 4, à l'exception de l'atome d'hydrogène, caractérisé en ce qu'on fait réagir un composé de formule générale IV

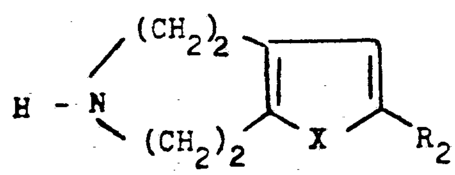

(IV)

dans laquelle $R_2$ est défini comme dans la revendication 1, avec un composé de formule générale (Va)

$$R_{1a}\text{—}Z \qquad \text{(Va)}$$

dans laquelle
$R_{1a}$ est défini comme dans la revendication 4 à l'exception de l'atome d'hydrogène et
Z est défini comme dans la revendication 1,
et si on le désire ensuite, on transforme un composé obtenu de formule générale (Ia) dans laquelle $R_{1a}$ représente un groupe aralcoyle avec 7 à 9 atomes de carbone éventuellement substitué dans le noyau aromatique par un atome d'halogène, au moyen de nickel de Raney en présence d'un alcool correspondant en un composé correspondant de formule générale (Ia) dans laquelle $R_{1a}$ représente un groupe alcoyle avec 1 à 12 atomes de carbone ou
on transforme un composé obtenu de formule générale (Ia) dans laquelle $R_2$ représente un groupe alcoxycarbonyle avec en tout 2 à 4 atomes de carbone, au moyen d'une hydrolyse en un composé correspondant de formule générale (Ia) dans laquelle $R_2$ représente un groupe carboxy ou on transforme un composé obtenu de formule générale (Ia) en ses sels, en particulier en ses sels physiologiquement supportables avec un acide ou une base minéral(e) ou organique.
Priorité : 18.02.1981 (DE-31 05 858)

7. Procédé selon les revendications 1 à 6, caractérisé en ce que la réaction est effectuée dans un solvant.

8. Procédé selon les revendications 1, 4 et 7, caractérisé en ce que la réaction est effectuée en présence d'une base et à des températures entre 0 et 100 °C, de préférence cependant à des températures entre 0 °C et la température d'ébullition du mélange réactionnel.

9. Procédé selon les revendications 2, 5 et 7, caractérisé en ce que la réaction est effectuée en présence d'un acide et à des températures augmentées, de préférence à la température d'ébullition du mélange réactionnel, par exemple à des températures entre 80 et 100 °C.

10. Procédé selon les revendications 3, 6 et 7, caractérisé en ce que la réaction, dans le cas où Z représente un groupe partant nucléophile, est effectuée en présence d'une base et à des températures entre 0 et 150 °C, de préférence cependant à des températures entre 20 et 80 °C ou, dans le cas où Z représente un groupe hydroxy, sous azote et en présence d'un catalyseur d'hydrogénation à des températures entre 80 et 250 °C, de préférence à des températures entre 100 °C et la température d'ébullition de l'alcool mis en œuvre, ou, lorsque Z ensemble avec un atome d'hydrogène de l'atome de carbone voisin représente un atome d'oxygène, avec de l'hydrogène activé catalytiquement à des températures entre 80 et 250 °C, de préférence cependant à des températures entre 100 et 200 °C.